(19)

**Europäisches Patentamt**
**European Patent Office**
**Office européen des brevets**

(11) **EP 4 607 536 A1**

(12) **EUROPEAN PATENT APPLICATION**
published in accordance with Art. 153(4) EPC

(43) Date of publication:
**27.08.2025 Bulletin 2025/35**

(21) Application number: **23868227.2**

(22) Date of filing: **20.09.2023**

(51) International Patent Classification (IPC):
**G16H 50/80** (2018.01)

(52) Cooperative Patent Classification (CPC):
**G16H 50/80**

(86) International application number:
**PCT/JP2023/034161**

(87) International publication number:
**WO 2024/063106 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.09.2022 JP 2022149414**

(71) Applicant: **Sawatari, Ryusuke**
**Tokyo 101-0025 (JP)**

(72) Inventor: **Sawatari, Ryusuke**
**Tokyo 101-0025 (JP)**

(74) Representative: **Lippert Stachow Patentanwälte Rechtsanwälte Partnerschaft mbB Frankenforster Strasse 135-137 51427 Bergisch Gladbach (DE)**

(54) **INFECTION COUNTERMEASURE METHOD AND INFECTION COUNTERMEASURE SYSTEM**

(57) [PROBLEM]
To estimate the Infection Situation of a group at lower cost or at an earlier stage, and to implement Infection Control for the group using that information.

[SOLUTION]
It comprises Observation Units that collect Propagating Substances (X) within Regions Ri and measure their amount, and Analysis Units that calculate values of one or more Indicators, or their approximations, for the Infection Countermeasures of the entire Area, based on the measured values (VXi) of the amount of the Propagating Substances. The Analysis Units output Countermeasures for the Infection Countermeasures for the entire Area based on the calculated values of the said Indicators or their approximations.

Figure 1: Functional Block Diagram of Infection Countermeasure System 1

## Description

[FIELD OF THE INVENTION]

The present disclosure relates to methods and systems for implementing infection countermeasures.

[BACKGROUND OF THE INVENTION]

Countermeasures to protect individuals and groups from pathogen infections (hereinafter referred to as "Infection Countermeasures") are a common concern for human beings, regardless of time and place. Infection Countermeasures include those aimed at individuals as well as those aimed at groups, and various methods have been developed thus far.

[PRIOR ART REFERENCES]

[PATENT LITERATURE]

[PTL 1]

Japanese Unexamined Patent Application Publication No. 2020-008969

[SUMMARY OF THE INVENTION]

[PROBLEMS TO BE SOLVED BY THE INVENTION]

Infection Countermeasures aimed at individuals include prevention and treatment. Key issues for these include preventing pathogens from entering the body as much as possible and minimizing damage if they do enter. On the other hand, for Infection Countermeasures aimed at groups, the key issues include preventing pathogens from entering the bodies of the group through the propagation of pathogens as much as possible, and preparing medical systems for when infections expand within the group.

While various methods have been developed for Infection Countermeasures aimed at individuals, Infection Countermeasures for groups face many challenges, possibly due to the rarity of events such as pandemics that require such measures. For example, understanding the situation of the infection within a group (such as how many infected people there are and the rate at which the infection is expanding or shrinking; hereinafter referred to as the "Infection Situation") is essential for taking countermeasures. However, it requires enormous costs (expenses, effort, time, legal regulations, etc.). For example, even if everyone is tested for the presence of infection simultaneously at a single point in time, that information becomes outdated the moment the tests are completed. In other words, even if there are people afterward who newly become infected or recovered from infection, it cannot be known without conducting the tests again. Additionally, there is the issue that people who have just become infected may not be detected in tests due to the small amount of pathogens present in their bodies.

The present disclosure addresses the problem of estimating the Infection Situation of a group (including estimations through approximation) at lower cost or at an earlier stage, and implementing Infection Control within the group using that information. Here, Infection Control typically means improving the Infection Situation, such as by reducing the number of infected people. However, it also includes responding accordingly to the Infection Situation, such as strengthening medical systems or adjusting economic activities. The term "Infection Countermeasures" will be used hereinafter to refer to estimation and control in this sense.

[MEANS FOR SOLVING THE PROBLEM]

To solve the above problem, the present disclosure utilizes what is referred to as Propagating Substances contained in the environment (hereinafter assumed to be in the air, but other media such as water are also possible). Propagating Substances refer to substances that suggest the presence of an infection source (such as infected people) nearby, with a typical example being the pathogen itself. For example, a person with a cold emits Propagating Substances into the environment through sneezing, coughing, speaking, breathing, etc. Therefore, if Propagating Substances are present in the environment, there is a high possibility that an infection source is nearby. Not only that, but it also suggests the possibility that people may become newly infected by allowing pathogens to enter their bodies (hereafter referred to as "taking in" or "taking into"), for instance, by coming into contact with the infection source.

The present disclosure estimates the presence of infected people within the target region, the number of new infections in that region, etc., by observing (collecting and measuring) Propagating Substances in the environment, thereby aiding in Infection Control. Moreover, not only by measuring Propagating Substances, but also by combining this with the amount of people in that region, more detailed estimates are conducted. Note that, although the present disclosure deals with a group of people, the methods presented can also be applied to groups of animals, plants, or the like. Additionally, as a special case, it can also be applied when the group consists of a single individual.

Specifically, the Infection Countermeasure System according to the present disclosure is characterized by comprising:

> an Observation Unit configured to collect a Propagating Substance (X) within a certain arbitrary region Ri and measure the quantity thereof; and
> an Analysis Unit configured to calculate one or more Indicator values or approximate values thereof for Infection Countermeasures of the entire Area, based on the measured value (VXi) of the quantity of the Propagating Substance.

In particular, the Analysis Unit is characterized by inputting a Residence Quantity (si), which represents the cumulative duration of stay of a group within the region Ri, estimating a Suspension Quantity (qi) of the Pathogen from the measured value (VXi) of the quantity of the Propagating Substances, calculating the Emission Quantity (ei) or the Absorption Quantity (ai) of the Pathogen using the said Suspension Quantity (qi) and the said Residence Quantity (si), and outputting Countermeasures for Infection Countermeasures of the entire Area, which are pre-associated with the said region Ri, based on the said results of the calculation. The Emission Quantity (ei) of the Pathogen is obtained based on a result of dividing the Suspension Quantity (qi) by the Residence Quantity (si). The Absorption Quantity (ai) of the Pathogen is obtained based on a result of multiplying the Suspension Quantity (qi) by the Residence Quantity (si).

Here, the Suspension Quantity (qi) of the Propagating Substances includes cases where it is proportional to the quantity of Propagating Substances (VXi)-the Relative Suspension Quantity q'. Similarly, the Residence Quantity, Emission Quantity, and Absorption Quantity include the Relative Residence Quantities (si', si''), the Relative Emission Quantity (ei'), and the Relative Absorption Quantity (ai'), respectively. Note that proportionality also encompasses approximate proportionality.

Furthermore, the method for implementing Infection Countermeasures according to the present disclosure is characterized by estimating or predicting the Infection Situation by collecting Propagating Substances in the environment and measuring their quantity as a measured value VXi. Note that the results of estimating or predicting the Infection Situation may also be utilized for control purposes. The same applies in the following descriptions.

Here, the target Area includes locations that are away from the place where the Propagating Substances are collected, by at least several tens of meters or any distance targeted by known techniques at the time of filing of the present disclosure.

The Infection Situation is estimated or predicted by measuring the quantity of substances in the environment that are in states of both causes and effects of deterioration in the functions of living organisms (for example, viruses in wastewater are excluded).

Specifically, the Infection Countermeasure Method according to the present disclosure is characterized by:

including a step of collecting Propagating Substances (X) within a certain region Ri and measuring their quantity;
a step of calculating one or more Indicator values or approximate values thereof for Infection Countermeasures for the entire Area, based on the measured value (VXi) of the quantity of the Propagating Substances;
and a step of outputting Countermeasures for Infection Countermeasures for the entire Area, based on the calculated values of the said Indicators or approximate values thereof.

Additionally, the method according to the present disclosure is characterized by estimating or predicting Indicators representing the Infection Situation using at least one of VXi, si', or si''.

And, the method according to the present disclosure is characterized by calculating $ei'=VXi/si'$ using VXi and si'.

And, the method according to the present disclosure is characterized by calculating $ai'=qi'*si''$ using VXi and si''. Note that the Influence Quantity, such as the number of newly infected people pi, may be estimated based on ai' obtained through the calculation.

And, the method according to the present disclosure is characterized by calculating $ai'=ei'*si'*si''$ using ei', si', and si''.

And, the method according to the present disclosure is characterized by calculating e0'' by integrating the measured values from multiple Collection Units.

And, the method according to the present disclosure is characterized by calculating $a'=ei'*s'*s''$ or $a'=e0''*s'*s''$ using ei' or e0'' from locations with similar Infection Situations, even for regions R without a Collection Unit.

And, the method according to the present disclosure is characterized by measuring qi', ei', and ai' to assess the effectiveness of Infection Countermeasures. Additionally, these values may be used to optimize the Infection Countermeasures through feedback control.

According to the system and method for implementing Infection Countermeasures of the present disclosure, even when the number of newly confirmed people is decreasing, a lockdown can be implemented if ai' is increasing. Conversely, even when the number of newly confirmed people is increasing, the lockdown can be lifted if ai' is decreasing. The ability to make predictions using ai' in this way is a feature of the present disclosure.

The method described above can be realized as a system for implementing Infection Countermeasures. Furthermore, it can be realized as a computer program for operating this system or device.

[EFFECTS OF THE INVENTION]

According to the present disclosure, it is possible to estimate the Infection Situation of a group at lower cost or at an earlier stage, and to implement Infection Countermeasures for the group using that information.

[BRIEF DESCRIPTION OF THE DRAWINGS]

[FIG. 1] A functional block diagram of an Infection Countermeasure System 1 in the present disclosure.

[FIG. 2] A configuration diagram when the Analysis Unit 3 is implemented by a program.

[FIG. 3] An example of Area 6 with one Collection Unit 21.

[FIG. 4] An example of Area 6 with multiple Collection Units 21.

[FIG. 5] An explanatory diagram of the Decision Criteria 41.

[FIG. 6] A flowchart of the Decision Process 1 (Embodiment 1).

[FIG. 7] A flowchart of the Infection Quantification Function 1 (Embodiment 1).

[FIG. 8] A flowchart of the Infection Quantification Function 2 (Embodiment 2).

[FIG. 9] A flowchart of the Decision Process 3 (Embodiment 3).

[FIG. 10] A flowchart of the Infection Quantification Function 3 (Embodiment 3).

[FIG. 11] An explanatory diagram of the Update Criteria 42.

[FIG. 12] A flowchart of the Update Process (Embodiment 4).

[FIG. 13] A flowchart of the Infection Quantification Function 4 (Embodiment 4).

[FIG. 14] The relationship between the number of newly infected people and the number of newly confirmed people.

[FIG. 15] A flowchart of the Infection Restriction Process (Embodiment 8).

[FIG. 16] A diagram explaining an advantage of Embodiment 2.

[DETAILED DESCRIPTION OF THE EMBODIMENTS]

Below, the various Examples and Embodiments of the present disclosure will be explained with reference to the drawings. Note that, although the present disclosure primarily discusses the process invention, it can also be easily applied to the creation of devices, programs, and recording media that store the said programs, which have similar functions.

[Example 1]

Although the central idea of the present disclosure is simple, to the inventor's knowledge, there are no countries or regions that have implemented Infection Countermeasures based on a similar idea. This seems to be evidence of the difficulty in reaching that idea. The inventor conducted research using his own body for approximately 2 years, through observation and recording, and realized that the idea was effective. Note that regarding the inventor's constitution, refer to the supplement at the end of the present Example.

In the following, the outbreak of the novel coronavirus SARS-CoV-2 (hereinafter referred to simply as the "virus"; however, when there is a risk of confusion with viruses in general, it will be referred to as "coronavirus"), which began in early 2020 in Japan, will be divided into waves from the 1st to the 7th, each corresponding to the following periods: the 1st wave, from early January to early May 2020; the 2nd wave, from mid-May to mid-October 2020; the 3rd wave, from late October 2020 to mid-February 2021; the 4th wave, from late February to late May 2021; the 5th wave, from early June to late November 2021; the 6th wave, from early December 2021 to mid-June 2022; and the 7th wave, from late June to August 7, 2022 (In the Examples of the present disclosure, the observation results until August 7, 2022 (Sun) are shown). Note that the division of each wave may vary depending on perspective, but it is not particularly important for the following discussion. Furthermore, compared to the fluctuations in the number of newly confirmed people, the above divisions may appear slightly earlier. This is because the divisions are based on fluctuations in the number of newly infected people (which, as will be described later, fluctuate 1 to 2 weeks earlier) rather than the statistics of newly confirmed people. Note that "newly infected people" refers to those newly infected by taking in the pathogen on a given day, while "newly confirmed people" refers to those newly diagnosed as positive and reported in the statistics on that day (Be aware that not all newly infected people necessarily appear as newly confirmed people in the statistics later, as some may recover before being tested, for example).

Additionally, as it is preferable to be aware of holidays when interpreting the observation results, they are listed below. The holidays in 2020, including Sundays, were January 1 (Wed), January 13 (Mon), February 11 (Tue), February 23 (Sun), February 24 (Mon), March 20 (Fri), April 29 (Wed), May 3 (Sun), May 4 (Mon), May 5 (Tue), May 6 (Wed), July 23 (Thu), July 24 (Fri), August 10 (Mon), September 21 (Mon), September 22 (Tue), November 3 (Tue), and November 23 (Mon).

Furthermore, the holidays in 2021, including Sundays, were January 1 (Fri), January 11 (Mon), February 11 (Thu), February 23 (Tue), March 20 (Sat), April 29 (Thu), May 3 (Mon), May 4 (Tue), May 5 (Wed), July 22 (Thu), July 23 (Fri), August 8 (Sun), August 9 (Mon), September 20 (Mon), September 23 (Thu), November 3 (Wed), and November 23 (Tue).

Moreover, the holidays in 2022, including Sundays, were January 1 (Sat), January 10 (Mon), February 11 (Fri), February 23 (Wed), March 21 (Mon), April 29 (Fri), May 3 (Tue), May 4 (Wed), May 5 (Thu), July 18 (Mon), August 11 (Thu), September 19 (Mon), September 23 (Fri), October 10 (Mon), November 3 (Thu), and November 23 (Wed).

Additionally, when referring to the "7-day average" of the number of newly confirmed people below, it will not mean the usual "average of the current day and the previous 6 days," but rather "the average of the current day and the 3 days before and after." The reason is that the former is a value that smooths out the number of newly confirmed people and delays it by 3 days, while the present disclosure seeks to observe only the smoothed value without delay.

<The Main Idea of The Present Disclosure>

The idea of the present disclosure is based on two findings. Details will be provided later, but here a summary will be presented. First, the coronavirus can be detected over a much wider range than generally believed, which is the first finding. This means that by simply examining the amount of virus in the air within a narrower region, one can determine the amount of virus emitted collectively by a larger number of people as a group. Then, what can we do once we know the amount of virus emitted collectively by a group? It would be reasonable to think that "the amount of virus is correlated to the current total number of infected people within the group, so it can be estimated." On the other hand, the assertion that "the amount of virus is correlated with the number of newly infected people arising from the group (likely on the same day), and thus future statistical predictions can be made" may seem absurd at first glance (at least to the inventor). However, in reality, the latter is more accurate, which is the second finding.

For another point, while it is just a remark rather than a finding, in adjacent locations (such as neighboring prefectures or towns) there is a tendency for the Infection Situation for the virus to be similar (probably due to the transmission of the virus among people traveling between these locations, the similarity in human activities and weather conditions, etc.). For example, the trend in the number of newly confirmed people in Tokyo and Kanagawa Prefecture is very similar.

From the above 3 points, we reach the following idea. That is, "by simply examining the amount of virus in the air within a narrower region, it is possible to estimate the number of newly infected people in a wider region (likely on that day) and predict the number of newly confirmed people in the future." Here, the estimation and prediction of newly infected people and newly confirmed people includes not only the numbers themselves but also, more broadly, the estimation and prediction of increases and decreases in these numbers (specifically, the comparison of the numbers at two different points in time). Moreover, in Infection Countermeasures, it is not always necessary to estimate and predict both the number of newly infected people and the number of newly confirmed people. For example, predicting the number of newly confirmed people is useful for purposes such as securing hospital beds in advance based on that information. However, estimating the number of newly infected people is more important for making decisions about lockdowns at an early timing, for instance.

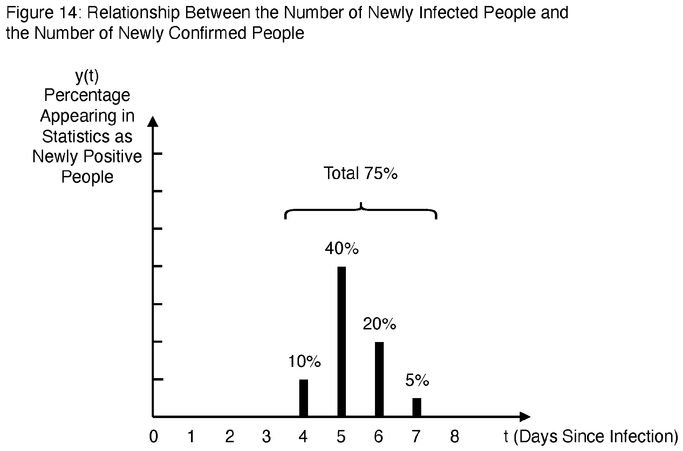

Note that, in the present Example, since statistical data on the number of newly infected people was not available, only the prediction of the number of newly confirmed people is presented. However, the number of newly confirmed people is a secondary quantity that manifests from the past number of newly infected people, and it can essentially be interpreted as an estimation of the number of newly infected people (probably the newly infected people on that day, though this is not certain; there may be a few days of shift). Naturally, the fluctuations in the number of newly infected people and the number of newly confirmed people are quite similar, and a slightly more detailed explanation is provided below. As schematically illustrated in Figure 14, the number of newly confirmed people has a distribution such that "the percentage of people who test positive on day t after infection is y(t) percent of the newly infected people." (The figure shows a case where y(4)=10%, y(5)=40%, y(6)=20%, y(7)=5%, and y(t)=0 for other values of t. As mentioned earlier, the sum of y(t) for all t does not necessarily equal 100%, and in this example, it is set at 75%. Additionally, actual statistics related to the coronavirus are published by organizations such as the National Institute of Infectious Diseases.) In other words, the number of newly confirmed people is a delayed occurrence of past newly infected people, where it is smoothed out by performing a weighted summation of the counts from several days before and after. (However, in reality, as will be discussed later, statistical fluctuations due to factors such as the day of the week or whether it is a holiday are added, making this relationship more complex.) Below, details on the two findings mentioned above will be provided.

<Details of the First Finding>

First, let's discuss the first finding. In general, it is said that there is a risk of infection with the coronavirus in indoor spaces or on trains with infected people, in places adjacent to rooms with infected people where air circulates through gaps around doors, or outdoors when approaching an infected person, and so on. Naturally, the virus (whether it is infectious or not is a separate matter; for example, it could be remnants or fragments of the virus. Hereinafter, the term "virus" will be used in this sense) can propagate even to places distant from these. However, for example, in places at the seaside about 10 meters from the sea surface, if there are only a few people within a 30-meter radius, there is generally no need to worry about the presence of the coronavirus. However, in reality, even in such places, during periods of infection spread, there are cases where it can be detected by the human body. The detected virus is likely a mix of virus emitted by people who were present at the location both currently and in the past, virus emitted and propagated from people in distant places, and virus emitted and leaked from people inside nearby buildings or vehicles. (Even in a room with tightly closed windows, virus can enter from the outside, though in small amounts. Similarly, it is assumed that the virus also leaks inversely, meaning from indoors to outdoors.)

<Details of the Second Finding: The Relationship between the Day of the Maximal Number of Newly Confirmed People and the Amount of Virus>

Next, moving on to the second finding. The inventor actually walked around Zone A in Kanagawa Prefecture almost every day during the 3rd, 6th, and 7th waves, observing and recording the amount of coronavirus in the air. As a result, there were a total of 5 days when the amount of virus was extremely large (therefore, the observation was discontinued, considering the burden on the body) and notably larger compared to the most recent days. Each of them is described below. First, during the 3rd wave, the amount of virus peaked on December 24, 2020 (Thu), and was notably large. As described later, during the 3rd wave, it was considered that there was a correlation between the amount of virus in the air and the number of newly confirmed people in Tokyo 14 days later. In fact, on January 7, 2021 (Thu), which is 14 days after December 24, the number of newly confirmed people in Tokyo was 2,520 (initially announced as 2,447 but later corrected), which was the maximum during the 3rd wave. Note that Tokyo, rather than Kanagawa Prefecture, was used for comparison because the prefectural boundaries are merely an artificial designation; geographically, there is a significant amount of movement between Zone A and Tokyo; the Infection Situations in both areas are similar as previously mentioned; and Tokyo has a larger sample size and wellestablished system as the capital, making data such as daily changes the most reliable. Next, during the 6th wave, the amount of virus peaked on January 26, 2022 (Wed), and was notably large. As described later, during the 6th and 7th waves, there is a strong possibility of a correlation between the amount of virus in the air and the number of newly confirmed people in Tokyo 8 days later. The maximum number of newly confirmed people in Tokyo was 21,562 (initially announced as 21,576 but later corrected) on February 2, 2022 (Wed), which was 7 days later. Finally, during the 7th wave, there were several days with a notably large amount of virus, in descending order: (1) July 26, 2022 (Tue), (2) July 21, 2022 (Thu), and (3) July 25, 2022 (Mon). (However, the difference between (2) and (3) was slight.) Then, 8 days after (1), on August 3, 2022 (Wed), the number of newly confirmed people in Tokyo was 38,940, which was maximal, but it was the second largest of the 7th wave, not the maximum. The actual maximum number of newly confirmed people was 40,406 on July 28 (Thu), which was 7 days after (2).

Note that regarding the use of the term "extremely large" above, the amount of virus on December 24, 2020, during the 3rd wave, was extremely large at the time, but it may have been smaller compared to the 6th and 7th waves. However, comparison is difficult due to the time gap between the observation periods.

Additionally, in the present disclosure, location names are represented by symbols. First, A and A1 are zones within Kanagawa Prefecture (Zone A1 is located within Zone A). Next, P1 to P10 are locations within Zone A.

On the other hand, during the 4th wave, the peak could not be identified because, for example, observations were simplified or spaced out due to concerns about the highly infectious Alpha variant; the fluctuations in the amount of virus exhibited complex movement (Although the relationship is unclear, the 4th wave coincided with a period when the proportion of the Alpha variant increased and vaccination started in Japan. The trends in the proportion of variants in Tokyo and nationwide can be referenced respectively at https://www.bousai.metro.tokyo.lg.jp/_res/projects/default_project/_page_/001/013/947/49ka i/2021061008.pdf and https://www.nikkei-science.com/?p=64775. Additionally, the vaccination status nationwide in Japan, though not specific to Tokyo, can be checked at: https://info.vrs.digital.go.jp/dashboard/.); and there were no days with a notably large amount of virus among those observed. (The largest amount of virus during the 4th wave among those observed was on April 6, 2021 (Tue), although it was not considered notably large, followed by May 11 (Tue) to May 13 (Thu).) Next, during the 1st wave, no observations were conducted because we were fundamentally unaware of our own constitution. Furthermore, during the 2nd wave, while it was predicted on August 1, 2020 (Sat) that the peak in the number of newly confirmed people would pass within 2 weeks, the idea of the present disclosure had not yet been reached at that time, and active observation was not conducted, so this is only for reference. Finally, during the 5th wave, observations were conducted intermittently and no predictions were made due to concerns about the highly infectious Delta variant, as well as the fact that, conversely, there was almost no virus detected from around October 2021 after the peak (until late December, when the 6th wave began).

<Details of the Second Finding: The Relationship between Finer Fluctuations of the Virus and the Number of Newly Confirmed People>

Regarding the 2nd and 3rd waves, as well as the early part of the 4th wave, the details of finer fluctuations will be described later in <Details of the Present Example>. Furthermore, regarding the period from the mid-stage of the 4th wave to the 7th wave, they will be explained in Examples 2 to 4.

<Details of the Second Finding: Summary>

From the results above and described later, it can be said that the increase and decrease in the amount of virus tends to manifest as a delayed increase and decrease in newly confirmed people. Additionally, an approximate relationship is suggested, where when the amount of virus is similar, the number of newly confirmed people that manifests after a delay will also be similar. By utilizing this characteristic, it is possible to predict the future number of newly confirmed people based on the amount of virus, or estimate the current number of newly infected people, allowing for early Infection Control. Note that the term "approximate" is used here for the reasons such as the following. First, it would be more accurate to consider the amount of people in addition to the amount of virus (for example, according to the interpretation in Embodiment 2, the quantity obtained by multiplying the amount of virus by the amount of people has a stronger correlation with the number of newly confirmed people). Second, other than the amount of people, factors such as the proportion of people with antibodies or those currently infected, the proportion of variants in the amount of virus, and the weather conditions on that day (temperature, humidity, wind flow, etc.) are also relevant. Third, as mentioned earlier, when the amount of virus manifests as the number of newly confirmed people, the distribution shown in Figure 14 is also relevant. Fourth, there are biases in statistics and aggregation (such as fewer tests conducted on holidays) and errors.

Let us supplement the first finding here. It seems that a sufficient amount of virus disperses in the air in the community to be detected by a human body, even in the initial stages when the number of newly confirmed people is very low. For example, the inventor first detected a variant that was prevalent during the 5th wave (presumably the Delta variant or a strain within the B.1.617 lineage to which it belongs) on May 24, 2021 (Mon), while observing outdoors under Plan 2 described later. While moving around that day, they noticed that the bodily sensation when taking in the virus was different than usual, but without giving it much thought, they ended up taking in a lot of it, resulting in diarrhea and frequent urination later that night. (The inventor had never experienced frequent urination before, but experienced it a few times only during the period up until around September 2021, and has not experienced it since.) The number of people infected with the B.1.617 lineage in Japan at that time was still low. According to an announcement by the Ministry of Health, Labour and Welfare on May 21, 2021, the cumulative number of people infected with this lineage detected at quarantine stations from overseas by May 7 was 160 (https://www.mhlw.go.jp/stf/new page_18805.html). Additionally, according to an announcement by the Ministry on May 26, 2021, the cumulative number of people infected with this lineage within Japan by May 24 was only 29 (https://scienceportal.jst. go.jp/newsflash/20210527_n01/ and https://www.fu kuishimbun.co.jp/articles/-/1325735). That is, even if statistical data on the number of newly confirmed people are not available or insufficient, it seems possible to estimate the Infection Situation and implement Infection Control at an earlier stage, by simply measuring the amount of virus in the air.

Now, the details of the present Example will be explained, starting with the method for specifically observing (collecting, and determining or measuring the amount) the amount of virus.

<Details of the Present Example: Method for Observing the Amount of Virus (Collection)>

First, in general, the amount of coronavirus in the air tends to be larger in places with more people than in places with fewer people. Additionally, the amount changes daily but also fluctuates within a single day. The most notable characteristic is that it is smaller in the morning and larger in the evening and beyond. The cause is unclear, but for example, the virus in the bodies in a group may decrease during sleep as physical strength is restored, while it may proliferate after waking due to fatigue and so on, and possibly transmit among people during the day through contact. The cause is unclear, but for example, the virus in the bodies in a group may decrease during sleep as physical strength is restored, while it may proliferate after waking due to fatigue and possibly transmit among people during the day through contact. The inventor conducted observations for about 60 minutes a day (in reality, the amount of virus for that day can generally be assessed in approximately 10 seconds to 30 minutes; however, they conduct longer observations to increase accuracy. Additionally, even without actively observing, they automatically sense the amount of virus in the air through breathing during simple movements or other tasks, which can add up to 2 hours or more). Considering the above, the locations and time frames were varied into three plans (hereinafter referred to as Plans 1 to 3) according to the outbreak of infections. The reason for not using a single plan is that when the number of infected people is low, the virus can only be detected in crowded places or enclosed spaces. Conversely, when the number of infected people is high, it becomes difficult to assess the fluctuation in the amount of virus in such places, and it is easier to assess by investigating locations and time frames where the virus can be detected only when the number of infected people is high. We will explain in order, starting from the period when the number of infected people is low. (Just to be sure, this does not mean these locations have a particularly large amount of virus. If the amount of people is the same, all locations are considered the same.) Plan 1 covers various buildings and facilities in Zone A1, as well as outdoor areas, starting from around 18:00 to 19:00. Plan 2 covers the streets from Point P1 to Point P2 to Point P3 and the nearby roads, in addition to the locations from Plan 1, starting from around 18:00 to 19:00. (Note that not all of these locations are necessarily traversed each time. However, as mentioned later, the observation results do not vary significantly.) Plan 3 covers the streets from Point P4 to Point P5 (turning right and walking along the southwest sidewalk of the major street) to Point P6 (crossing to the northeast side of the major street here) to Point P7 to the three-way intersection ahead (specifically, at Point P7, turning left once and circling around a nearby block for about 10 minutes before reaching the three-way intersection) to Point P8 to Point P9 to Point P3 to Point P10, and the nearby roads, starting from around 16:00 to 17:00. (Again, not all of these locations are necessarily traversed each time.) Additionally, common to the three plans mentioned above, regardless of the time frame, the amount of virus was also referenced as needed in visited locations, inside trains traveling to other areas in Kanagawa Prefecture or towards Tokyo, and inside the home when entering from outdoors while at home during periods of infection spread.

When observed under the same plan, the amount of virus fluctuates daily but also changes depending on the location. However, the distribution of the intensity of the virus levels tends to follow the same general trend even on different days, and does not change randomly from day to day. Therefore, even if the observation time is reduced to, for example, 30 minutes, the amount of virus at the remaining locations can generally be guessed, so the observation results do not change significantly. Even if they do, it would only be by about one in levels (L0 to L4) described later. Note that the scale of the distribution by location of the amount of virus varies from small to large, but when it is small, it may be described as "the XX intersection has more than the surrounding area" if it is outdoors.

Although it is a very rough guideline, Plan 1 is easiest to observe when the number of newly confirmed people (predicted in the future) is up to several hundred, Plan 2 when it is from several hundred to several thousand, and Plan 3 when it exceeds several thousand. In the present Example, during the 2nd wave, observations were conducted using to Plan 1 (however, active observations did not start until around early August). During the 3rd wave, observations were conducted using to Plan 1 until the virus was first detected outdoors in late November 2020, after which the scope was gradually expanded to Plan 2 over a period of about two months. During the 4th wave, Plan 2 was primarily used, but observations were also conducted using Plan 1 when the amount of virus was small. During the 5th wave, Plan 2 was primarily used, and in mid-August 2021, near the peak, observations were also conducted using Plan 3. In contrast, from October 2021, when the virus was hardly detected outdoors, observations were conducted using Plan 1. During the 6th wave, observations were initially conducted using Plan 1 or Plan 2, but after January 18, 2022, observations were conducted using Plan 3. During the 7th wave, observations were conducted using only Plan 3.

Although it has not been implemented, when measuring with a machine, collection devices such as an air sampler with a gelatin filter could be used. Following any of the above plans, it would be sufficient to collect air while moving along the same route for about 30 minutes at a fixed time each day. For example, one could leave Point P2 at 18:30, walk a bit slowly, and arrive at Point P3 at 19:00. If the amount of virus collected is insufficient, it would be sufficient to extend the time, lengthen the route, operate multiple collection devices in parallel, or change the plan (or, collect and measure in parallel using all three plans and select the measurement results from the appropriate plan according to the Infection Situation). Then, the RNA of the virus attached to the filter is extracted and the copy number is determined, for example, through PCR. However, care is required because the amount of virus collected with different plans is not mutually comparable. Note that the amount of virus can change even by simply moving to a neighboring street. Additionally, on larger streets, the situation can vary depending on whether one walks on the left or right side. Therefore, when walking the "same route," it is preferable to walk on the "same side of the same street." Additionally, it is better if the amount of people along the route is as even as possible. This is because if one passes through exceptionally crowded areas, the amount of virus collected there will dominate, making the fluctuations in the amount of virus collected from other locations relatively small and difficult to reflect in the measurement results. Similarly, since the amount of virus tends to be larger indoors than outdoors, care is required when both are passed through in a single collection. For example, if the average amount of virus is measured in advance for each location and the route is planned to pass through locations with no significant difference in the amount, it can prevent the amount from becoming dominant in any specific location. Alternatively, it is considered that collecting with a fixed device in a single high-traffic location could also be effective (for example, installing it on the ceiling of a subway station ticket gate in Zone A1).

<Details of the Present Example: Method for Observing the Amount of Virus (Determination of the Amount)>

The amount of virus was determined by two methods (referred to as the "Level Method" and the "Comparison Method," respectively). Until the middle of the 4th wave (April 19, 2021), the Level Method was primarily used for determination, but the Comparison Method was occasionally used in conjunction, and in rare cases, only the Comparison Method was used. After that, the Comparison Method was used for determination. The reason for changing the determination method was that the main objective of the observation was shifted to capturing the maximal and minimal points of fluctuations in the amount of virus, and the Comparison Method was more suitable for tracking finer fluctuations. Additionally, during the 5th, 6th, and 7th waves, observations were also conducted using Plan 3, which was significantly different in terms of location and time frame than before, making it confusing to record using the same scale. In the following, we will explain each in turn.

First, in the "Level Method," the amount of virus on the day was determined in 5 levels, from Level 0 to 4 (hereafter referred to as L0 to L4). L0 indicates that no virus or an extremely small amount is detected. L1 indicates a small amount of virus, and as the numbers increase, the amount of virus detected also increases. Note that these levels are determined subjectively based on the body's response, so the criteria are highly likely to fluctuate over the long term.

Next, in the "Comparison Method," the amount of virus is compared with that at a past point in time to determine whether it is larger, smaller, or about the same. Here, the past "point in time" refers to a specific day or week, typically the previous day, 7 days prior (the same day of the previous week), or the entire previous week. As an application of this, comparisons can also be made between two different points in time (for example, A and B). If the amount of virus is larger than at point A but smaller than at point B, it may be determined to be an intermediate amount. Records in the Comparison Method are kept in free-form text rather than symbols, as in the Level Method. Note that simply comparing the amount of virus detected using different plans is meaningless, so this is not done. However, even if the plans are different, the amounts detected under partially overlapping conditions (the same time and location, or adjacent time frames and locations where a similar amount of virus can be detected, even if not exactly the same) are compared, provided that the range of the overlap is sufficiently large.

The advantage of the Comparison Method is that it can track finer fluctuations compared to the Level Method. For example, the amount of virus on a certain day might be L2, and the following day it might also be L2, but slightly larger than the previous day. While both would be recorded as L2 in the Level Method, the Comparison Method allows for recording the difference as well. On the other hand, the advantage of the Level Method is that it can compare the amount of virus on two arbitrary days, though not as finely as the Comparison Method (excluding cases where the two days are far apart and the criteria are likely to have fluctuated).

Note that, in the Level Method, the determination results of D1, D2, and F were exceptionally recorded. The inventor initially considered these as "levels" of the amount of virus, but it gradually became clear that they should not be treated as equivalent. However, due to this history, they are recorded as levels. The details will be explained below.

First, D1 and D2 indicate that the detection of "particularly concentrated virus" (a strong reaction occurring suddenly in the throat, to the point where the voice becomes hoarse, which does not occur in levels L0 to L4) occurred once and twice on the same day, respectively. When this "particularly concentrated virus" was detected, D1 or D2 was recorded, regardless of the amount of virus (L0 to L4 or F described later) in other locations on the same day. The distinctive feature of D1 and D2 is that, while the amount of virus typically does not change significantly with just a few meters of movement, the detection of D1 and D2 is sudden, and moving just a few meters away from the location prevents the detection. Additionally, it has only been detected indoors or on trains. The likely cause is that, at the time or not long before, an infected person was nearby, and the inventor inhaled that person's exhalation. Note that, as described later, from the start of recording on August 1, 2020, until August 7, 2022, D1 was detected 4 times, D2 was detected 7 times, and D3 or higher (i.e., detection of "particularly concentrated virus" 3 or more times in one day) was never detected.

Next, F is a classification that was used only during the early stage of observation (from the start of recording on August 1, 2020, until December 2020). It was recorded 9 times, but has not been used since. During the early stage of observation, the virus was generally only detected in limited areas indoors or on trains with high population density. By visiting these scattered locations, the amount was comprehensively judged and recorded as levels L0 to L4. However, on some days, small amounts of the virus were unexpectedly detected, even in sparsely populated locations, though indoors. Initially, it was unclear what this meant, so it was specially recorded as F. However, it was later realized that F was simply a form of daily fluctuation in the amount of virus at the same location, and from then on, it was incorporated into the previously mentioned levels L0 to L4, no longer being used as a standalone record. That is, the virus can be detected not only in places with high population density but also everywhere (even this fact was not initially obvious). Just as its amount fluctuates daily in places with high population density, it also fluctuates daily in sparsely populated places. And the virus is usually too low to be detected in sparsely populated locations, and F is just a recognition of days when it increased enough to be detected. This alone should not be considered a reason for special treatment. Initially, any such detection was recorded as F, regardless of the amount of virus in other locations. However, in December 2020, both the level detected in other locations and F were recorded twice, indicating some slight fluctuation in the criteria. However, F is not particularly important in the following predictions.

To ensure accuracy, a more detailed account will be provided. Initially, since the body's reactions stronger than L4 only occurred upon detecting "particularly concentrated virus," D1 and D2 were treated as "levels" L5 and L6, respectively. However, on December 24, 2020, when the amount of virus reached its largest during the 3rd wave, the amount exceeded the preceding L4, and no "particularly concentrated virus" was detected. This indicated that it was not appropriate to label D1 as L5. However, since rewriting past records was not an option, it was recorded as L4 out of necessity, with a note stating that "the amount of virus was extremely large." However, as there were no similar occurrences since then (it seems that this is related to changing the observation locations to areas with a smaller amount of virus as the infection spread; and additionally, at the peaks of the 6th and 7th waves mentioned earlier-specifically on January 26, 2022, and July 21, 25, and 26-the amount of virus was also extremely large, but these were recorded only using the Comparison Method), D1 and D2 are treated as levels L5 and L6 in the records.

<Details of the Present Example: Details of Observations and Predictions (Introduction)>

In the following, the results of each observation and prediction will be described. Just to be sure, the inventor has not conducted any statistical analyses of, for example, the effective reproduction number. Additionally, for people today who can look back on the progression of the Infection Situation since the first case was found in Japan in January 2020 as a confirmed past, the predictions in the present Example may appear to simply trace the fluctuations. However, it should be emphasized that, naturally, the inventor made predictions not based on hindsight, but simply knowing the number of newly confirmed people up to that day, even without knowing whether it would increase or decrease afterward (in terms of the graph, there is no information to the right of that day). Note that while some of the predictions in the present Example were recorded after midnight on that day, the content was determined within the same day and will be explained as recorded on that day. Additionally, the predictions described below list all, even those that are more akin to records than predictions or those that were inaccurate. However, although smaller fluctuations were also observed and recorded beyond those described here, any that could not be interpreted as predictions, such as those indistinguishable from errors, have been excluded.

<Details of the Present Example: Details of Observations and Predictions (Delay in the Number of Newly Confirmed People during the 2nd and 3rd Waves)>

We will describe the correlation between the amount of virus and the number of newly confirmed people 14 days later during the 3rd wave mentioned earlier. First, although it is not strictly the same as the prediction based on the "amount of virus" in the air (L0 to L4 in the Level Method), which is the central idea of the present disclosure, the prediction based on "particularly concentrated virus" D1 and D2 will be described to explain the 14-day delay. However, since the detection of D1 and D2 cannot be expected to be accurate as described below, the predictions should be considered for reference only. Furthermore, it should be noted that the value of 14 days is not universal and may vary due to factors such as the existence of variants with different incubation periods and the accessibility of testing (for example, in Japan, many people did not get tested unless they had a fever lasting more than four days until the situation changed around May 2020).

The inventor began recording the amount of virus in the air from August 1, 2020, during the 2nd wave (although not every day), but did not believe from the outset that the number of newly confirmed people could be predicted based on the amount of virus. Initially, they noticed that there tends to be an increase in the number of newly confirmed people about 2 weeks after detecting D2. Whether an individual inventor passes near infected people while moving depends on chance, so whether D1 and D2 is detected or not is largely influenced by probabilistic factors (however, it is considered that the possibility of the spread of locations with "particularly concentrated virus" is higher for D2 than for D1). Furthermore, the detection of "particularly concentrated virus" would approximately double in frequency if the time spent indoors or on a train doubles. However, since such precision was not initially considered necessary, only the number of times was recorded, regardless of whether the time spent outdoors was longer or shorter (in contrast, with the detection of the "amount of virus," even if the inventor's time spent outdoors doubles, only the accuracy of judgment changes, and the amount does not double). Due to the reasons above, it should be noted that the accuracy of the predictions is not high. Furthermore, for some reason, the amount of virus is not particularly large on the next day after detecting D2.

Specifically, D2 was detected on August 9, 2020 (Sun), September 2 (Wed), September 9 (Wed), September 10 (Thu), October 1 (Thu), October 22 (Thu), and December 7 (Mon) during the 2nd and 3rd waves. On the other hand, there were no detections of D1 or D2 from the 4th wave onward. (This may be related to factors such as increased time spent observing outdoors from the 4th wave onward and increased vaccination in Japan. For reference, the dates when D1 was detected are December 29, 2020 (Tue), February 23, 2021 (Tue), May 16 (Sun), and September 5 (Sun). Initially, it was hoped that the number of new confirmed people would increase approximately 2 weeks after detecting D1. However, it seems that D1 detection alone is strongly influenced by chance factors and may not be useful for predictions.) The reason for including not only the dates but also the days of the week is that the number of new confirmed people exhibits characteristics for each day of the week. It is said that due to the lower number of tests on Sundays and holidays, there is a tendency for fewer new confirmed people on the following day (such as on Mondays). As a result, part of the number of new confirmed people may appear with a delay of two days or more, or they may recover while waiting for tests, resulting in not being reflected in the statistics. Therefore, when the 14th day after detecting D2 coincides with these days, it becomes difficult to determine whether the number of new confirmed people has increased or not.

Based on the above records alone, it is difficult to conclude that the detection of D2 is effective for predictions. However, from the inventor's perspective, as of September 2 mentioned above, it was thought that the 2nd wave would subside. Therefore, they found it interesting that it began to expand again about a week later and began to consider whether the detection of D2 might be effective for predicting an increase in the number of newly confirmed people. Particularly, the detections on October 1 and October 22 mentioned above were highly impressive, as exactly 14 days later (October 15 (Thu) and November 5 (Thu)), there was a sharp increase exceeding 250 cases, which made them pay attention to the number "14 days". (To reiterate, when looking back as a confirmed past, predictions may seem easy by following the fluctuations, as the number of newly confirmed people has periodic ups and downs during this period. However, that is hindsight, and at the time of detection, all future outcomes were uncertain. Note that the results of the detections on September 9 and September 10 were considered difficult to appear in the statistics because, 14 days later, September 23 (Wed) and September 24 (Thu) fell right after a long holiday. They may have appeared as a sharp increase in the number of newly confirmed people on September 26 (Sat), but it remains unclear.) Regarding the detection on December 7, 2020, the infection had already spread, and there were consecutive days with over 300 newly confirmed people. The situation was different from the preceding times when D2 was detected (from August 9 to October 22, 2020), making it difficult to assess how the number of newly confirmed people would be 14 days later, on December 21, 2020 (Mon). In the end, it was predicted that the number would increase, but that turned out to be incorrect.

By the way, in the observations of the 2nd, 3rd, and 4th waves, detections of D2 or a sharp increase in the amount of virus and the subsequent sharp increase in the number of newly confirmed people 14 days later often occurred on Thursdays for some reason. Some literatures interpret that "there are days in the week when the aggregation process of newly confirmed people clusters, and as a result, the number of newly confirmed people on Thursdays tends to increase" (for example, https://www.asahi.com/articles/ASNBY7316NBPUTIL01B.html). However, that interpretation alone does not explain why there are some Thursdays with notable numbers and others without. Additionally, in the present Example, sharp increases other than on Thursdays are also predicted, such as on March 3, 2021 (Wed), which will be described later. However, if this interpretation from the literature is a contributing factor, then if there are no days when the aggregation process clusters, the delay of "14 days" could shift forward or backward, or have a range such as "13 to 15 days".

<Details of the Present Example: Details of Observations and Predictions (3rd Wave)>

From here on, we will return to predictions based on the "amount of virus." The inventor was unable to predict the increase in the number of newly confirmed people over approximately 2 weeks from November 7, 2020 (Sat) (until around November 21 (Sat), when 539 cases were recorded) when the infection expanded further (the cause of the increase remains unclear, but it may be related to the replacement of the predominant strain in the country from B.1.1.284 to B.1.1.214 around the same period). The reason the increase was not predicted is that past increases in the number of newly confirmed people following the detection of D2 had been transient; and no significant changes in the amount of virus were detected during the 14 days prior to this period-specifically from October 24 (Sat) to around November 7 (Sat). However, on November 16 (Mon), prompted by the fact that 3 out of the most recent 4 days were L0, when reviewing the amount of virus over the most recent 14 days (from November 3 (Tue) to November 16 (Mon)), it appeared subjectively to be nearly flat. Looking at the records, it seems to be nearly constant excluding minor fluctuations, with many L0s (Levels L2, L3, L4, and F are scattered among the many L0s). At that time, the central idea of the present disclosure had not been clearly reached, and it was believed that if there were many L0 days, the number of newly confirmed people would decrease. Therefore, a prediction was made, presenting two possibilities: that "the number of newly confirmed people would decrease over the next 14 days (from November 17 (Tue) to November 30 (Mon)), and that there was also a possibility that it might remain somewhat flat." The results seemed nearly flat, excluding fluctuations by day of the week. While the prediction for the period from November 7 to November 30 has the drawback of not anticipating an increase to the extent exceeding 500 in the number of newly confirmed people as described earlier, the inventor began to consider the hypothesis that not only predictions based on D2 but also those based on the amount of virus-specifically, that "if the amount of virus is similar, the number of newly confirmed people 14 days later will also be similar"-upon observing this result.

Note that the virus was first detected outdoors in late November 2020 (having only been detected indoors or on trains prior to that), and since then, the amount of virus outdoors has also been incorporated into observations.

Subsequently, on December 3 (Thu), the amount of virus increased by another level, reaching its highest level in the most recent month. However, since predictions based on the amount of virus were still at the hypothesis stage, it was predicted somewhat cautiously that "the number of newly confirmed people 2 weeks later may increase by another level or so." In fact, the number of newly confirmed people on December 17 (Thu), 14 days later, sharply increased to 822, which was also the highest number in the most recent month. This suggests that not only D2, but also a sharp increase in the amount of virus can manifest as a sharp increase in the number of newly confirmed people 14 days later.

Later, on December 24 (Thu), the amount of virus increased sharply to a level never detected before, leading to its recording as L4 with the note that "the amount of virus is extremely large" (the amount on this day was the largest among those observed during the 3rd wave). Furthermore, on the following day, December 25 (Fri), there was a significant decrease. At this point, with the infection spreading to an unprecedented extent and presenting a different situation than before, there was no certainty that the peak in the amount of virus (a sharp increase followed by a sharp decrease) could predict the peak in the number of newly confirmed people. So it was only recorded that "there is a possibility that the number of newly confirmed people will increase on January 7, 2021 (Thu)." In fact, the number of newly confirmed people also peaked on January 7, as described earlier.

Just for a reference, it was recorded on January 31, 2021 (Sun) that "the current number of infected people seems, subjectively, to be about the same as in mid-December 2020. The current number of infected people in Tokyo is also about the same as in mid-December." At first glance, it may seem like a tautology, but the first half indicates that the "amount of virus 14 days prior" for both "now" and "mid-December" is subjectively similar. On the other hand, "number of infected people" in the latter half indicates the "number of newly confirmed people." In other words, the purpose of the whole record was to confirm the hypothesis that 14 days after days with similar amounts of virus, the number of newly confirmed people would also be similar. However, it cannot be verified by comparing records such as levels (as it is difficult to make comparisons by checking the amount of virus recorded around January 17, 2021, and around the period from November 26 to December 6, 2020). Furthermore, since this record is a post hoc confirmation rather than a prediction, it is for reference only.

Additionally, on January 20, 2020 (Wed), it was recorded that "the number of infected people has remained high for about a week, not improving or worsening. It seems to have slightly improved in the last few days" (since the purpose was to record rather than predict, the expression is ambiguous). Note that "the number of infected people" here indicates the "number of newly infected people." That is, upon reviewing the amount of virus over the most recent week, this record summarizes that L4 was recorded from January 13 (Wed) to 15 (Fri), and L3 was recorded on January 17 (Sun), 19 (Mon), and 20 (Wed) (with no observations conducted on the 16th and 18th). However, looking at the number of newly confirmed people 14 days later, from January 27 (Wed) to February 3 (Wed), it shows a decrease compared to the most recent week. Although the main purpose of this text was to serve as a record rather than a prediction, it is difficult to say that it is correct as a prediction.

<Details of the Present Example: Details of Observations and Predictions (Early Stage of the 4th Wave)>

Next, we will discuss the turning point from the 3rd wave to the 4th wave. During the decline of the 3rd wave, the decrease in the amount of virus began to be observed around February 1, 2021, and during the period from February 12 (Fri) to February 21 (Sun), the amount of virus was particularly small (only L0 to L2 were recorded). However, on February 22 (Mon), it increased to L3, and after detecting D1 on February 23 (Tue), levels L3 to L4 continued until March 1 (Mon). Therefore, on February 23, it was briefly predicted that "the number of newly confirmed people might slightly increase on March 9 (Tue), 14 days later," and then, as described later, a more detailed prediction was made on March 8 (Mon). And in fact, 14 days after the period when the virus was particularly small (from February 26 (Fri) to March 7 (Sun)), the number of newly confirmed people was near its minimal point.

In the subsequent period from around March 2, 2021 (Tue) to April 10 (Sat), observations were switched to simpler methods, such as avoiding crowded places, due to concerns about the pathogenicity of the Alpha variant. There were days when observations were not conducted. Note that, since this period, records using the Level Method have become less frequent.

On March 3, 2021 (Wed), the virus further increased, and it seemed to be close to the amount during 14 days prior to when the number of newly confirmed people was around 400 to 500 in the past (although no records are available, it is thought to have been around early December 2020). Subjectively, it was judged to be closer to the time when the number was 500 rather than 400, leading to the prediction that "the number of newly confirmed people on March 17, 2021 (Wed), 14 days later, would be about 500." Although the result was 409, this was a highly accurate prediction based on human sensory perception, and strongly supports the hypothesis that the number of newly confirmed people 14 days later can be predicted based on the amount of virus. Note that the number of newly confirmed people exceeded 400 for the first time in about a month (since February 18 (Thu)), and the graph also shows a characteristic increase.

<Summary of the Present Example>

Detailed results of the observations and predictions regarding the 4th wave afterward and the 6th and 7th waves will be discussed in Examples 2 to 4. Here, a summary of the present Example will be provided. As described so far, although the method of observation and prediction based on human sensory perception is not rigorous, it is somewhat possible to predict the increase, decrease, peaks, and numbers of newly confirmed people in the entire Tokyo by simply examining the amount of virus in the air inhaled by an individual while walking.

In the present Example, no Infection Control was implemented. However, naturally, if estimation and prediction of the Infection Situation are possible, Infection Control, such as securing hospital beds earlier, is also possible. For example, when the prediction was made on March 3, 2021, that "the number of newly confirmed people on March 17, 14 days later, would be about 500," measures such as preparing treatment systems earlier could have been taken. Additionally, if measures to prevent infection spread had been taken around February 23, 2021, when the increase in the virus was detected, it would have been possible to prevent or delay the outbreak of the 4th wave. Furthermore, not only during periods of infection spread but also during periods of decline, it would be possible to implement control such as increasing the amount of going out earlier and resuming economic activities.

Additionally, being able to estimate and predict can also have a positive psychological effect on people. If estimation and prediction are not possible, even if it is understood after an increase in the number of newly confirmed people that "Infection Control should have been implemented 14 days ago," it is unclear "then, what specific actions should be taken now, either as a group or individually." This can lead to psychological passivity, making Infection Control more prone to being gradually loosened. In contrast, if estimation and prediction can be made early, there is a significant advantage in that what needs to be done now becomes clearer, allowing for active implementation of measures.

Furthermore, as mentioned earlier, the number of newly confirmed people is a secondary quantity that manifests from the past number of newly infected people with a delay. Thus, the present Example is essentially an estimation of the number of newly infected people. Then, why is it possible to make estimations based on the amount of virus? The first hypothesis is that the amount of virus in the air is largely composed of the virus emitted by most recent (that is, on the same day or a few days prior) newly infected people in the nearby area, and as a result, the number of newly confirmed people can be estimated based on the amount of virus. When a person becomes infected with the virus, the amount of virus in their body changes over time, following a course such as increasing from an initial low level to a peak after several days, and then decreasing again. Therefore, it can be considered that the virus in the air is largely composed of the virus emitted on the day when the amount of virus in the body of the most recent newly infected people peaked. If this hypothesis is correct, dividing the amount of virus in the air by the amount of people in the nearby area would yield a quantity that is approximately proportional to the number of newly infected people in that area. However, in the case of the coronavirus, it is said that the amount of virus in the body peaks approximately on the day of symptom onset (according to a study published on March 23, 2021, viral shedding peaks two days after symptom onset: https://www.amed.go.jp/news/release_20210323-02.html). Then, the 14-day delay observed during the 2nd and 3rd waves is roughly equal to the number of days from symptom onset to the announcement of the number of newly confirmed people, but it is somewhat longer compared to experiential sense.

The second hypothesis, which will be described in detail in Embodiment 2, is that the amount of virus in the air represents the number of newly infected people on the same day who take it in and become infected. Taking the detection of D1 and D2 described in the present Example as an instance, it can be inferred that on the day the inventors detected D1 and D2, there were other people who had also taken in the concentrated virus. Furthermore, some of them became infected due to that. And it can be said that the number of people was higher on the day D2 was detected compared to the day D1 was detected. Similarly, on days when the inventor detected a larger amount of virus, many other people in the nearby area were also exposed to a larger amount of virus, and among them, some accidentally took in a large amount of the virus (such as by coming close to an infected person) and became infected. And it can be said that the larger the amount of virus detected by the inventor, the higher the probability of infection, and the more the number of newly infected people increases (assuming the amount of people in the nearby area remains the same). If this hypothesis is correct and Embodiment 2 can be applied, multiplying the amount of virus in the air by the amount of people in the nearby area would yield a quantity that is approximately proportional to the number of newly infected people in that area. Based on this hypothesis, the estimation of the number of newly infected people in the present Example can be said to be an approximation that assumes a constant amount of people.

Other than that, there may be a more complex mechanism linking the amount of virus to the number of newly infected people, but it is uncertain at this point.

Note that the amount of virus in the air may decrease on rainy or windy days, but based on the accuracy of detection by the human body, no difference greater than daily fluctuations has been observed. However, there have been occasions when a decrease in the virus was observed during the time when fresh air was blowing in.

Here, a comparison with several existing technologies will be made. First, there already is a mechanism that measures the amount of PM2.5, photochemical oxidants, pollen, etc., in the air and issues alerts. These determine whether the target substances are detected to the extent harmful to the human body. In contrast, the method in the present disclosure is characterized by not questioning whether the amount of virus (or more generally, pathogens) detected is harmful to the human body. In other words, the focus is on the fluctuations of the virus rather than its absolute quantity. Even if the detected amount of virus is extremely small, it is indirectly estimated how many newly infected people there are in the surrounding area (in the present Example, the entire Tokyo). This characteristic is considered to be one of the obstacles to reaching the idea of the present disclosure (as Infection Countermeasures are generally concerned with the amount of virus that poses a risk of infection, and there is little need to accurately determine the amount of virus when there is no risk). Another obstacle is that while substances like PM2.5 in the air are a "cause" of harm to the human body, they are not an "effect." In contrast, viruses in the air are both a "cause" of harm to the human body (as the virus causes infection when it enters the body) and an "effect" (as it is emitted from infected people). Therefore, attention may be focused on the aspect of the "effect" that is not present in PM2.5, etc. For example, when the virus is present in small amounts, there is no concern about infection. Therefore, it is easier to focus solely on the aspect of the "effect," that is, the presence of infected people nearby, rather than the "cause." The second hypothesis described earlier and Embodiment 2 are characterized by focusing on the "cause" here. On the other hand, even when focusing on the "effect," it is not obvious whether it correlates with the "number of newly infected people" as in the first hypothesis, but rather it seems more natural to consider that it correlates with the "total number of infected people" at present.

Second, technologies have already been developed to measure the amount of coronavirus in wastewater and to estimate or predict the infection situation. This technology also focuses on the "effect" aspect rather than the "cause" aspect of the virus.

Third, while there may already be ideas for collecting air on vehicles like trains to determine the presence of viruses, it seems that they do not consider estimating the number of newly infected people based on the amount of virus.

<Supplement: Inventor's Constitution and Detection of Coronavirus by the Human Body>

The inventor is able to detect the presence of coronavirus (or its remnants) in the air, including its concentration. The indirect proof of this is the prediction in the present Example, but for themselves, it is an undeniable fact. (The inventor originally does not have particularly strong skin or mucous membranes, but believes that their sensitivity to the coronavirus due to their constitution may be the cause.) When the concentration of the virus is high, there is a distinctive sensation right upon inhalation (a feeling of a foreign substance passing through the airway, which was never felt before the coronavirus pandemic). Additionally, outdoors, if there is no wind, the concentration of the virus at the location can be sensed as a degree of discomfort in the back of the throat (likely due to inflammation, reaching a different area than the pain experienced with a common cold) after stopping and taking a few breaths. The discomfort can be felt to recover within a few tens of seconds if it is mild. When walking outdoors while paying attention to changes in this throat discomfort, it is often possible to notice changes in the concentration of the virus over a distance of about 10 to several tens of meters. For the inventor, the concentration of the virus can be sensed as if visualizing the density of smoke.

Naturally, the body's reactions mentioned above can also be interpreted as being caused by factors other than the virus. However, if the cause were, for example, environmental pollutants, it would be difficult to explain why the amount is larger indoors than outdoors (although this is not always the case, as it can vary depending on location and time of day). Conversely, if it were causative substances of sick building syndrome or the like, it would be unlikely to be detected in large amounts outdoors as well. Furthermore, it is natural to consider that a higher detection in crowded places is due to human-derived substances. Moreover, it has never been felt for such a long duration before the pandemic, and if the intensity of the reaction correlates with the outbreak, it is most reasonable to consider it to be due to the coronavirus.

Furthermore, different variants may elicit different responses in the body upon inhalation, and if the amount is moderate, it may be possible to distinguish between those variants. For example, when a large amount of the variant predominant during the 5th wave (presumably the Delta variant or a strain within the B.1.617 lineage to which it belongs) is taken into the body, it can cause diarrhea and frequent urination. Additionally, when a large amount of the variant predominant during the 4th wave (presumably the Alpha variant) is taken into the body, reactions may appear in areas such as the left chest, left armpit, and left foot. The variant predominant during the 6th wave (presumably the Omicron variant) also exhibited similar reactions at the initial stage to those observed during the 4th wave.

However, the inventor was not confident from the outset that they could detect the coronavirus. The origin lies in when they caught a cold in February 2020 (presumably due to an infection with the coronavirus). High fever persisted for nearly a week, and even after the fever subsided, their throat discomfort persisted for months. For example, there were moments when they suddenly entered a state of detecting a "particularly concentrated virus" while speaking. The inventor initially believed that the cause was the virus remaining in the throat without disappearing. However, after the state of emergency measures implemented in areas such as Tokyo and its vicinity on April 7, 2020, were lifted on May 25 and the number of people going out increased, the throat condition, which had temporarily improved, began to frequently worsen while going out. Particularly, it often worsened inside the stores they visited. This can be explained by the increase in the number of people and a significant rise in the amount of virus inside the stores. After that, they continued to observe the conditions under which their body reacted while going out, gradually losing any doubt that their throat condition was worsening in response to the virus in the air.

On August 1, 2020 (Sat), while the number of newly confirmed people in Tokyo and its vicinity was in the midst of increasing during the 2nd wave, approximately one week prior was a period for the inventor during which they felt almost no virus while going out. The viruses in the air are a mixture emitted by an unspecified number of people, so its smaller amount suggests that there should be fewer infected people also in the statistics (although at that point, it was believed that the amount of virus correlated with all infected people rather than newly infected people). If that were the case, they considered that the peak of the infection spread had already passed and kept a record. And the maximum number of newly confirmed people during the 2nd wave was 472 on the same day, August 1. This was the first prediction of the present Example.

[Example 2]

In the present Example, we will explain the observations and predictions during the mid to late stage of the 4th wave. Although the predictions in the present Example were often less clear or incorrect compared to those in Example 1 (the reasons are unknown, but it may be related to the increasing proportion of the Alpha variant, which has significantly different characteristics than before, as well as the widespread vaccination), all predictions will be documented for the sake of fairness.

<Details of Observations and Predictions (Mid to Late Stage of the 4th Wave)>

On March 8, 2021 (Mon), upon reviewing the amount of virus over the most recent 14 days, there was an increase during the period from February 22 (Mon) to February 28 (Sun) (strictly, to March 1 (Mon)) compared to the previous week, as described earlier. After that, including the previously mentioned March 3, the amount of virus further increased, but the amount of virus in the few days leading up to March 7 (Sun) and on March 8 was less than on March 3. Therefore, it was predicted that "this week (March 8 to March 14) would slightly increase and next week (March 15 to March 21) would increase further. After that (from March 22 onward), it would settle down (the expression 'settle down' is vague, but it means 'decrease')." The result is as follows. First, the prediction for "this week" can be considered correct, as described earlier in the background. Next, the prediction for "next week" is also close to correct when looking at the graph of newly confirmed people; however, the prediction for "after that" is incorrect. However, in hindsight, based on the observations of the amount of virus, the period referred to as "after that" should have been "the few days leading up to and including March 22" rather than "from March 22 onward." That is, if it had been predicted that "next week (from March 15 to March 21) would increase around Wednesday, but the few days leading up to March 22 would settle down," it is considered to be closer to correct.

On March 17, 2021 (Wed), the amount of virus was the largest since March 3 and similar to that level, so it was predicted that "14 days later, the week including March 31 (around March 29 to April 4) would have a similar number of newly confirmed people as this week (around March 15 to March 21), which is 14 days after March 3." Since the result showed an increasing trend rather than being similar, it is difficult to say it was correct (furthermore, although in hindsight, predicting the latter half of the week based only on the amount of virus in the first half of the week was also a mistake). However, the number of newly confirmed people on March 31 (Wed) was 414, which was almost the same as the 409 on March 17 (Wed). Additionally, on March 19 (Fri), one day later, the amount of virus was somewhat smaller than on the 17th, but was still similar. On April 2 (Fri), 14 days later, the number of newly confirmed people was 437 (initially announced as 440 but later corrected), which was also similar to the 414. Note that, on March 18 (Thu), in the middle, the amount of virus was significantly smaller than on the preceding and following days. However, 14 days later, on April 1 (Thu), the number of newly confirmed people was, conversely, higher than on the preceding and following days, at 475. This can be interpreted as an increase due to combining people infected on March 17 and tested positive 15 days later with those infected on March 19 and tested positive 13 days later.

On March 21, 2021 (Sun), upon reviewing the amount of virus over the most recent 14 days (from March 7 (Sun) to March 20 (Sat)), it was predicted that "this week (from March 21 (Sun) to March 27 (Sat)) would slightly decrease in the first half but increase again in the latter half. Next week (from March 28 (Sun) to April 3 (Sat)) would increase to the same level or more compared to last week (from March 14 (Sun) to March 20 (Sat))." The reasons are as follows. First, for the first week (March 7 to 13), the amount of virus around March 8 was not particularly large, but it subjectively increased afterward. Next, for the second week (March 14 to 20), it was based on the previously mentioned prediction recorded on March 17 and was subjectively adjusted by the amount of virus later between March 18 and 20 (for example, March 19 also had a similarly large amount as on the 17th). The results are as follows. For the "this week" portion, it is difficult to verify because changes within one week have fluctuations by day of the week. However, it can be said that the number of newly confirmed people for the week as a whole shows an increasing trend, making it half correct. For the "next week" portion, it is correct in the sense that there was an increase. However, it was unexpected that the degree of increase was larger than subjective assessment.

On April 6, 2021 (Tue), it was recorded that "the amount of virus is very large and increasing with each passing week" (since the primary purpose was to record rather than predict, the expression is vague). In hindsight, this day had the largest amount among those observed during the 4th wave. However, due to factors such as skipping some observations, there was no recognition at that time that the virus amount was at its peak. Furthermore, while it can be said that the number of newly confirmed people over the following 14 days was "increasing with each passing week," the number of newly confirmed people on April 20, 2021 (Tue) was 710 (initially announced as 711 but later corrected), which was not the maximum during the 4th wave.

In the following period, around April 11 (Sun) to May 16 (Sun), 2021, the amount of virus exhibited complex fluctuations. Furthermore, the predictions made during that period were incorrect. The reasons are unknown, but it may be related to the replacement of the predominant virus with the Alpha variant. (According to statistics from Tokyo, the percentage of N501Y variants, including the Alpha variant, which had been below 10% before, sharply increased to about 30% from the week of March 29, 2021 (Mon). Furthermore, it sharply increased to approximately 60% from the week of April 19, 2021 (Mon):
https://www.bousai.metro.tokyo.lg.jp/_res/projects/default_project/_page_/001/013/860/47kai/2021052708.pdf.) Moreover, the incorrect predictions during this period, conversely, highlight the uniqueness of the accurate predictions or similar results made in other periods. Below, we will explain each prediction and its result.

During the period from April 11 (Sun) to April 18 (Sun), 2021, the amount of virus decreased significantly (14 days later is from April 25 (Sun) to May 2 (Sun)). Therefore, on April 16 (Fri), it was predicted that "the infected cases would decrease rapidly, peaking next week (until April 24, Sat). If the incubation period of the Alpha variant is short, it will start to decrease a little earlier." However, the number of newly confirmed people continued to increase from April 25 to May 2, making the prediction incorrect.

Note that there was a long holiday from April 29 (Wed) to May 5 (Wed), 2021 (although April 30 (Thu) was a weekday). It seems that the significant decrease in the number of newly confirmed people around May 5 (Wed) is due to the reduction in the number of tests during the holiday.

From around April 19 (Mon), 2021 (14 days later is May 3 (Mon)), the amount of virus began to increase again. Therefore, on April 21 (Wed), it was predicted that "it would shift to an increase from May 3." It is difficult to verify this prediction as it assumes a decrease in the number of newly confirmed people until May 2, and the statistics are considered to have irregularities caused by the holiday, as mentioned earlier. However, it is hard to say that it is correct (looking at the numbers alone, it is incorrect).

On April 30 (Fri), 2021, upon reviewing the most recent amount of virus, it was recorded that "the amount of virus during the last week and this week (from April 19 (Mon) to April 30 (Fri), 2021) was nearly flat." This is not an explicit prediction, but if interpreted as meaning that "the number of newly confirmed people from May 3 (Mon) to May 14 (Fri), 14 days later, will also remain flat, excluding fluctuations by day of the week," it is hard to say it is correct (similar to the previous prediction, it is difficult to verify, but looking at the numbers alone, it is incorrect).

Additionally, the amount of virus during the period from May 11 (Tue) to May 13 (Thu), 2021, was second to April 6, the largest during the 4th wave. (Note that signs of an increase were observed starting around May 9 (Sun).) Later, on May 16 (Sun), having detected D1 as mentioned earlier, it was predicted that "the number of newly confirmed people would largely increase from May 23 (Sun) to May 30 (Sun), 14 days after the signs of increase were observed." However, in reality, there was a significant decrease, so it was incorrect. Additionally, on May 16, upon reviewing the amount of virus during the past week starting from May 2 (Sun), another prediction was subjectively made that "the number of newly confirmed people from May 16 (Sun) to May 23 (Sat) would remain nearly flat or slightly increase." It is difficult to verify because changes within one week have fluctuations by day of the week, but it is considered that there was a decrease, so this is also incorrect.

[Example 3]

In the present Example, we will explain the observations and predictions during the 6th wave. Note that no predictions were made during the 5th wave, as mentioned earlier.

<Details of Observations and Predictions (6th Wave)>

During the 6th wave, which began in early December 2021 and was dominated by the Omicron variant, observations focused on larger fluctuations, such as weekly changes, rather than finer daily changes. However, if there were days with characteristic amounts of virus, those were also recorded together. Note that, due to concerns about the highly infectious Omicron variant, observations were conducted intermittently until January 17, 2022. (After January 18, once the characteristics of the variant were understood, observations were made nearly every day.) Additionally, during the 6th wave, records were made using the "Comparison Method" rather than the "Level Method."

Regarding the "lag period" between the detection of the amount of virus and its reflection in the number of newly confirmed people, it was set at "14 days" during the 2nd, 3rd, and 4th waves. However, more than six months have passed since then, and there may have been changes in the ease of testing, the statistical system, and so on, compared to that time. However, the only information available was that the incubation period for the Omicron variant is approximately 3 days, which is about 2 days shorter than the approximately 5 days for the conventional strains (https://www.niid.go.jp/niid/ja/diseases/ka/corona-virus/2019-ncov/2484-idsc/10434-covid19-43.html). Moreover, even if there have been changes in the system, they were not considered significant, and the predictions for the 6th wave were initiated with an assumption of "12 days." In reality, although it cannot be clearly identified, upon reviewing the predictions and their results, "8 days" seems most likely.

Note that from April 29 (Fri) to May 5 (Thu), 2022, there was a long holiday (although May 2 (Mon) was a weekday, it is thought that many people took it off), and exactly "8 days later," from May 7 (Sat) to May 13 (Fri), there was an increase in the number of newly confirmed people (in the sense that the number was higher compared to the same weekdays of the previous week). In contrast, there was a decrease during the preceding and following periods. If this is due to the increase in going out during the holiday, the newly infected people during the long holiday can be considered to have appeared in the statistics as newly confirmed people 8 days after the infection. Even within the same 6th wave, circumstances changed between the peak period (January to February 2022) and the long holiday, such as the dominant Omicron variant shifting from BA.1 to BA.2. However, this result serves as one reference point for determining the lag period.

By the way, in Example 1, two hypotheses were presented as reasons for why the number of newly infected people can be estimated based on the amount of virus. When combining the first hypothesis-namely, the interpretation based on the "amount of virus emitted by newly infected people"-with the statistics from the long holiday, the "lag period" between virus detection by the inventor and the appearance of the number of newly confirmed people should be several days shorter than 8 days (i.e., the number of days until the amount of virus emitted after infection reaches its maximum, which, if equal to the incubation period, would be around 3 days), leading to a discrepancy with the results described below. This supports the second hypothesis-namely, the interpretation based on the 'amount of virus taken in by newly infected people'-rather than the first hypothesis.

In the 6th wave, based on the outbreak of the Omicron variant overseas and the fast increase in the number of newly confirmed people in Japan during the earliest days (until around early January 2022), it was assumed that the infection would rapidly spread. Therefore, rather than focusing on the finer fluctuations in the amount of virus, the observations were initiated with the main purpose of determining how long and to what extent it would increase. Additionally, on January 26, 2022 (Wed), the amount of virus was notably large (which could be described as extremely large) since the start of observations in the 6th wave, and the difference compared to the days before and after was also large. Therefore, on the following day, January 27 (Thu), it was predicted that "the number of newly confirmed people would reach its maximum on February 7 (Mon), 12 days after January 26." However, since testing in Tokyo was in a state of saturation-evidenced by the start of operations for "probable case (presumptive positive)" on January 29, 2022 (the Ministry of Health, Labour and Welfare announced the recognition of probable cases on January 24 (Mon); refer to, for example, https://www.tokyo-np.co.jp/article/159276)-clear results from the predictions were not expected to emerge. The actual number of newly confirmed people peaked on February 2 (Wed), 7 days later.

Next, on January 31, 2022 (Mon), although the amount was not as large as on January 26, it showed a sharp increase, leading to the prediction that the number of newly confirmed people on February 12 (Sat), 12 days later, will reach a smaller maximal than on February 7. The result was incorrect, as it conversely decreased for a Saturday. Additionally, since "12 days later" is a tentative figure, February 8 (Tue), which is "8 days after" the previously mentioned day, can be examined. Although it shows an increase compared to the previous day, it is difficult to verify as it is not distinguishable from the fluctuations by day of the week.

In the 6th wave, the rate of decrease in the amount of virus after the peak was more gradual than the rate of increase before the peak. Therefore, on February 11, 2022 (Fri), it was recorded that "the decrease in the amount of virus is gradual," and it was specifically predicted that the number of newly confirmed people would be "around just under 10,000," referring to from 9,000 to 10,000. (However, this was not about the number of newly confirmed people on a specific future date, but rather a prediction of the average around that time.

Additionally, at this point, it was highly likely that the previously mentioned "12-day" delay would be revised, so no specific number of days later was predicted.) The basis for this prediction was that, subjectively, the amount of virus around that day (around February 11) was slightly less than half of what it was around the peak of the 6th wave (around January 26). Assuming a proportional relationship between the amount of virus and the number of newly confirmed people, it was derived from the number of newly confirmed people around the peak (around 20,000). Although it is a very rough calculation, it is noted here as it was recorded. The result was that the number of newly confirmed people on February 19 (Sat), 8 days after the predicted date, was approximately 14,000 as a 7-day average-about 40-60% higher than predicted.

On February 15 (Tue), February 17 (Thu), and February 18 (Fri), 2022, the amount of virus increased compared to the previous week. Initially, it was thought to be an incidental increase and did not receive particular attention. However, noticing it continued except on February 16 (Wed), it was recorded on February 18 that "the amount of virus shifted to an increase from February 15." Furthermore, on February 20 (Sun), the amount of virus increased remarkably, reaching a level that was comparable to January 26, though not as large. On the following February 21 (Mon), there was a significant decrease, and it was recorded that "the amount of virus on February 20 was the second maximal in the 6th wave." It is difficult to verify these predictions. First, on February 23 (Wed), 8 days after the amount of virus shifted to an increase, the number of newly confirmed people increased significantly. (The difference from the number of newly confirmed people on the preceding and following days-February 22 (Tue) and February 24 (Thu)-was greater than the fluctuations by day of the week.) However, on February 28 (Mon), eight days after the second maximal, the number of newly confirmed people was higher compared to the preceding and subsequent days for a Monday, but it did not show a clear increase. Next, during this period from February 23 to 28, the 7-day average of newly confirmed people did not show a clear increase. However, since it has shifted from a decrease to a flat trend, it is considered that the increase in the amount of virus has been somewhat reflected in the statistics.

On February 27, 2022 (Sun), although the cause was unknown, the amount of virus was unusually small (the smallest since the peak of the 6th wave, and significantly reduced from the most recent few days), and could be considered zero compared to the most recent days. However, since this was reflected in the number of newly confirmed people in a smoothed manner, it was anticipated that there would be a day in the future with a significant decrease compared to the previous day, although it would not be zero. In fact, on March 7 (Mon), 8 days later, the number of newly confirmed people significantly decreased. This was a 42% decrease compared to the previous day, March 6 (Sun), and although Mondays tend to have lower numbers, it can be said that the extent was greater than fluctuations by day of the week. (Until then in 2022, the largest Monday decrease compared to the previous day had been 32% on February 21 (Mon).)

During the remaining period of the 6th wave, the amount of virus repeatedly increased and decreased, but there was no significant variation. Based on past experience, predicting the number of newly confirmed people during such periods is difficult, so only observations were conducted.

[Example 4]

In the present Example, we will explain the observations and predictions during the 7th wave. In the 7th wave, observations were conducted using Plan 3. Specifically, from July 7 (Thu) to August 7 (Sun), the start time for observations was from 16:25 to 16:50.

<Details of Observations and Predictions (7th Wave)>

In the 7th wave, the amount of virus began to continuously increase around the beginning of July. Then, on July 13 (Wed), the amount of virus was the largest since March of the 6th wave (though still less than on January 26, 2022), and the difference from the preceding and subsequent days was also significant. Although the "lag period" during the 7th wave was unknown, it was assumed to be close to the most likely "8 days" from the 6th wave. Therefore, it was predicted on the following day, July 14 (Thu), that "July 21 (Thu) would be the provisional peak for the number of newly confirmed people during the 7th wave." However, on July 21 (Thu), the amount of virus further increased (reaching an extremely large amount comparable to January 26), and the difference from the preceding and following days was also significant. Therefore, on the following day, July 22 (Fri), the provisional prediction was updated, and it was predicted that "July 29 (Fri) would be the provisional peak for the number of newly confirmed people." Although it seemed to be coming to an end, on July 25 (Mon), the amount of virus increased significantly again compared to the previous day, reaching nearly the same level as on the 21st, or slightly less. Then, on July 26 (Tue), the amount of virus increased further, surpassing the level on the 21st, and on the following day, July 27 (Wed), it decreased significantly. Therefore, on the 27th, the provisional prediction was updated, and it was predicted that "August 3 (Wed), which is 8 days after the 26th, would be the provisional peak for the number of newly confirmed people." Since then, until August 7 (Sun), no amount of virus surpassing that of July 26 was detected.

We will verify the above predictions. First, regarding the first provisional prediction, the actual maximal number of newly confirmed cases was 34,995 on July 22 (Fri), which was one day later than predicted. Next, regarding the second provisional prediction, the actual maximal number was 40,406 on July 28 (Thu), which was one day earlier than predicted. Then, regarding the last provisional prediction, although the maximal number was 38,940 on the predicted August 3 (Wed), it was about 4% lower than that of July 28. Ultimately, the maximum number of newly confirmed people during the 7th wave was on July 28. Additionally, regarding the increase in the amount of virus on July 25, the day before the last provisional prediction, the number of newly confirmed people 8 days later, on August 2 (Tue), was 30,842, showing no particular increase.

[Embodiment 1]

First, terms common to all embodiments are defined. For notation, x*y denotes multiplication, x/y denotes division, exp(x) represents the exponential function with the base of Napier's constant, $X \cap Y$ denotes the intersection of sets, and X U Y denotes the union of sets. Furthermore, for values denoted by a final letter of lowercase i or j (e.g., VAi, ej, ei'), i and j are assumed to denote subscripts unless otherwise specified.

<Area 6>

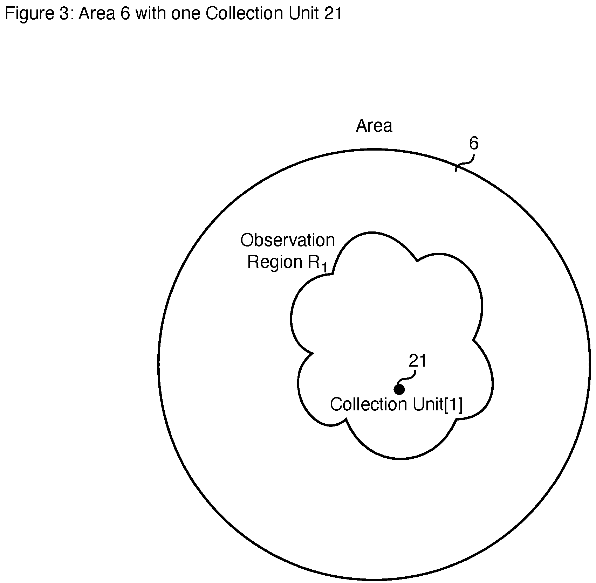

In the present disclosure, Infection Countermeasures are implemented for a group of people in a region referred to as "Area 6." (Figure 3. It may be a city block, a city, a prefecture/state, or a country. It generally has a three-dimensional extent and may move or change shape over time. Examples of movement include trains and ships.)

Generally, non-residents of Area 6 also enter and exit this Area 6. Whether or not one lives there is irrelevant to infection by pathogens. Therefore, it is preferable for the Infection Countermeasures in Area 6 to include non-residents in the targeted group, as long as they have spent time within Area 6.

<Infection Situation and Infection Status>

The state of infection with a pathogen within a group is referred to as the "Infection Situation" of the group. Similarly, the Infection Situation of a group in a specific region may also be referred to as the Infection Situation of that region. It is generally difficult to fully grasp the Infection Situation, and only partial information is available.

Additionally, the state related to the infection of an individual is referred to as their "Infection Status." This refers to conditions such as whether or not the individual is infected and, if so, how much they are emitting Propagating Substances (typically the pathogen itself) from their body.

<Pathogen and Pathogen Type>

There are multiple types of pathogens, such as the influenza virus and the tuberculosis bacterium. Additionally, even within the same influenza virus, there are generally multiple subtypes, such as H1N1 and H5N1. In the following, types of pathogens and subtypes within the same type will collectively be referred to as "Pathogen Types." For example, the Pathogen Type "influenza virus" includes lower-level Pathogen Types such as "H1N1" and "H5N1."

Similarly, although "influenza virus" and "tuberculosis bacterium" are distinct pathogen types, when considering the higher-level Pathogen Type of "pathogen," both are classified as lower-level Pathogen Types within it.

In the present disclosure, the routes by which pathogens are transmitted from person to person are not particularly limited. Examples include airborne transmission, droplet transmission, and contact transmission.

<Propagating Substances and Propagating Types>

The "Propagating Substance" of a pathogen with a specific pathogen type X (referred to as "Pathogen X") is a substance that suggests the presence of a source of infection for X nearby. Generally, there are multiple types for a single Pathogen Type. For example, these may include the pathogen itself, remains or remnants of the pathogen (denatured forms or parts of the pathogen), substances emitted by the pathogen in general, substances emitted by infected people in general, and even denatured forms of these substances while dispersed.

When a certain Propagating Substance is present in the air, there are instances where it is desirable to distinguish its forms, even if it remains the same substance. For example, it is generally said that pathogens have different infectivity depending on whether they are in the state contained in droplets or in the state of droplet nuclei. Distinguishing between these in Infection Countermeasures is meaningful.

The differences of this Propagating Substance, in terms of its substance or form, are referred to as "Propagating Types." Additionally, a Propagating Substance that has Propagating Type X is referred to as "Propagating Substance X." The pathogen X itself is also a Propagating Substance and is represented by a Propagating Type of the same name, X.

For example, when pathogen X is suspended, to distinguish between its infectivity and inactivation, it can be represented by Propagating Types XL and XD, respectively. Similarly, to distinguish between the state contained in droplets and the state of droplet nuclei, they can be represented by Propagating Types Xd and Xa, respectively. Furthermore, for example, the state of inactivated droplet nuclei can be represented by Propagating Type XDa. At this time, XDa is a lower-level Propagating Type of XD.

Two Propagating Types may overlap with each other. In the above example, $XL \cap Xd$ is generally not an empty set. Additionally, a higher-level Propagating Type does not need to be classified exhaustively by lower-level Propagating Types. For example, regarding the earlier Propagating Types Xd and Xa, a pathogen X contained in raindrops may not be classified into either of them. Furthermore, there may be cases where the identical Propagating Substance Z corresponds to two different Pathogen Types X and Y. These can include substances emitted due to inflammatory responses when a pathogen invades the human body.

Note that it is sufficient to define only Pathogen Types and Propagating Types necessary for infection countermeasures, without the need to define all conceivable types. For example, for pathogen X, Propagating Types XL, XD, Xd, and Xa can be defined, while for pathogen Y, only YL and YD can be defined. Furthermore, for pathogen Z, it is acceptable not to define any Propagating Types (other than Z).

In the following, A, B, C, ... will be used to represent all types defined without distinguishing between Pathogen Types and Propagating Types. That is, A=X, B=XL, C=XD, D=Xd, E=Xa, F=XDa, G=Y, H=YL, I=YD, J=Z, and so forth. Additionally, the letters X, Y, and Z will be used to represent any Pathogen Type or Propagating Type.

<Infection Countermeasure System 1>

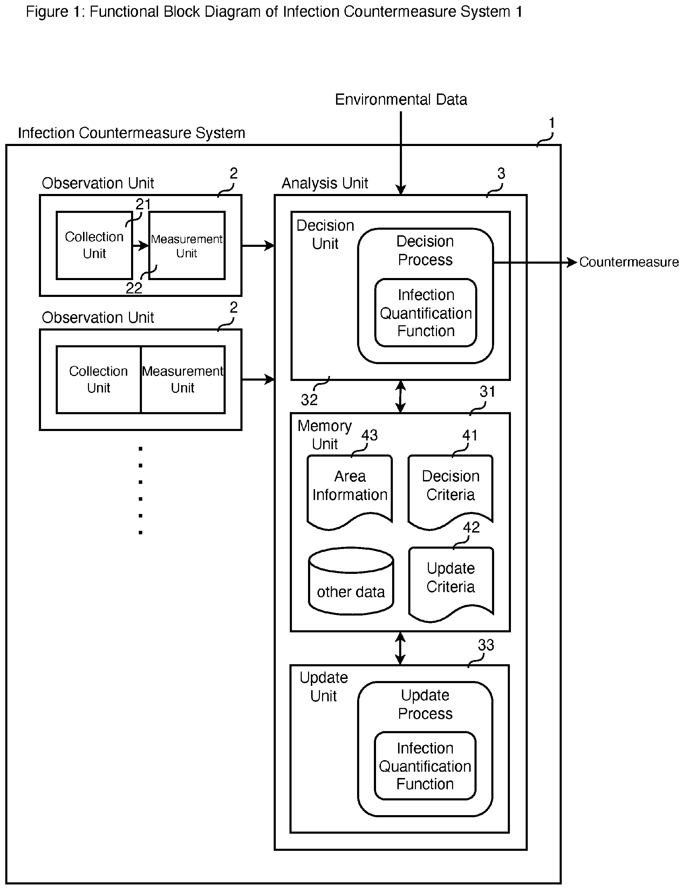

Figure 1 is a functional block diagram of the "Infection Countermeasure System 1." The Infection Countermeasure System 1 consists of one or more "Observation Units 2" and an "Analysis Unit 3."

<Observation Unit 2>

The Observation Unit 2, consisting of a "Collection Unit 21" that collects Propagating Substances and a "Measurement Unit 22" that measures the amounts of collected Propagating Substances, measures the amounts of Propagating Substances in the air.

The Collection Unit 21 and Measurement Unit 22 may be located in different physical locations. Additionally, there may also be cases where it is difficult or impossible to distinguish between the functions of collection and measurement. In such cases, an integrated unit of the Collection Unit 21 and Measurement Unit 22 can be considered (the second Observation Unit 2 from the top in Figure 1). As examples of such cases, the human body in Examples 1 to 4 can be cited. The human body can be viewed as having a respiratory system as a Collection Unit 21, with its surface area functioning as a Measurement Unit 22 due to inflammatory-like responses. However, it is more natural to consider it as an Observation Unit 2 in which the collection and measurement functions are integrated. Additionally, studies have reported training organisms such as dogs and honeybees to detect coronaviruses through odors, which can also be considered as an Observation Unit 2 that outputs observational results in the form of behavior. Furthermore, if the Propagating Substance absorbs light at a specific frequency, the amount of the Propagating Substance can also be measured by assessing the degree of absorption of that specific frequency component as the light travels a certain distance within Area 6. At this time, while no actual collection is conducted, it is considered that the Propagating Substance along the path through which the light has travelled have been collected.

<Collection Unit 21>

The Collection Unit 21 collects Propagating Substances in the air. An example configuration is to bring a device that combines a gelatin filter and a fan (with the fan serving to direct air onto the filter) into Area 6 as a Collection Unit 21, operating it for several hours to allow pathogens to adsorb to the filter. A Collection Unit 21 is assigned a number i starting from 1 (referred to as the "Collection Unit Number"), and a Collection Unit 21 with Collection Unit Number i is denoted as a "Collection Unit 21[i]."

The target Propagating Substances for collection can be either indoors and outdoors. Additionally, in addition to collecting from the air present at the location, it is also possible to directly direct the exhaled breath of people passing by onto the Collection Unit 21. The Collection Unit 21 itself can also be installed either indoors or outdoors. Additionally, it can be installed in a fixed position or used to collect while moving (for example, installed on the exterior or interior of cars, trains, airplanes, or ships). When installing in a location with strong winds, care is required to avoid situations where the amount of collected Propagating Substances varies due to being carried away by the wind, or where the amount of collected Propagating Substances fluctuates depending on wind direction, resulting in being prone to errors.

For cases such as when the amounts of Propagating Substances collected by a single Collection Unit 21 are small, or when it is desirable to avoid the effort of measuring for each of the many Collection Units 21 (for example, those installed on each floor or block of a building), the Propagating Substances collected by multiple Collection Units 21 can be measured as a single total amount. In this case, those multiple units are treated together as a single Collection Unit 21.

If the Propagating Substance is cultivable, the Collection Unit 21 may be equipped with a culturing function. This allows for an increase in the measured amount even when the amount of the Propagating Substance in the air is small, and prevents the degradation of the Propagating Substance when there is a delay between collection and measurement.

The Collection Unit 21 may conduct collection every day without any breaks, or it can collect only during specific time frames or days of the week. Furthermore, even when collecting for 24 hours a day, for example, there are different methods. Using a single filter, the total amounts of Propagating Substances for the day can be measured with the Measurement Unit 22. Alternatively, the filters can be exchanged every hour and passed to the Measurement Unit 22 as needed to measure the amounts of Propagating Substances for each hour.

There may be Collection Units 21 that collect only specific Propagating Types rather than all the Propagating Types being considered.

<Measurement Unit 22 and Measured Value>

The Measurement Unit 22 measures the amounts of Propagating Substances collected by the Collection Unit 21 and outputs the results. For example, the function to extract DNA/RNA from pathogens adsorbed on the previously mentioned gelatin filter and subject them to a qPCR device to measure the amount of pathogens.

The installation location of the Measurement Unit 22 is not restricted. The Collection Unit 21 may be placed within Area 6, and the amounts of Propagating Substances collected there can be measured by the Measurement Unit 22 located at a distant location. For example, after operating the previously mentioned Collection Unit 21 with a gelatin filter within Area 6 for a few hours, the filter can be removed and transported to a distant facility, where the amount of pathogens can then be measured using a qPCR device located there. In this case, for example, multiple Collection Units 21 may share a single Measurement Unit 22 in a time-division manner. In the present disclosure, whether the Measurement Unit 22 is shared or not is not important. Hereafter, we will explain assuming a one-to-one correspondence between the Collection Unit 21 and the Measurement Unit 22.

The quantity of the measurement results does not have to be the count of Propagating Substances itself. For example, it can be the Ct value from qPCR or a Boolean value indicating the "presence or absence" of the Propagating Substance (where "true" indicates "presence"). Alternatively, in the case of dogs that detect Propagating Substances by odor, it may be possible to have three levels consisting of strong reaction, weak reaction, or no reaction. Additionally, the area of colonies that appear on a Petri dish as a result of culturing pathogens for a certain period is also acceptable.

For example, in the case of an Observation Unit 2 that only determines the "presence or absence" of a Propagating Substance, as an advance preparation, it can be installed together with an Observation Unit 2 that accurately measures the amount of the Propagating Substance. By establishing a relationship such as "when the measurement result is true, the number of the Propagating Substance is N or more per cubic meter," the measurement results obtained as Boolean values can be converted into the amount of the Propagating Substance. Furthermore, by deploying such Observation Units 2 in multiple locations and obtaining a correspondence between the measurement results at each Observation Unit 2 and the amount of the Propagating Substance (for example, the percentage of deployed Observation Units 2 with measurement results of true) as an advance preparation, it is possible to measure the amount more accurately.

In the following, the measurement result will refer to a quantity equivalent to the count of Propagating Substances, such as the count itself, a quantity scaled by a constant multiple of the count, the count per unit time, or the count per unit volume.

The Measurement Unit 22 is assigned the same number i as the Collection Unit 21. Measurements are conducted for each Propagating Type, and the output of the Measurement Unit 22 is referred to as the "Measured Value," represented as VAi, VBi, VCi, ...

The measured value VXi is a sequence of numbers, and the amount of Propagating Substance X measured (hereinafter also referred to as detected) at time t (where t is an integer satisfying $t \geq 0$) is denoted as VXi[t]. The unit of time t is arbitrary, but here it is taken to be one day. That is, VXi[0] represents the amount of Propagating Substance X detected by Collection Unit 21[i] on the first day of Infection Countermeasures, and VXi[1] represents the amount on the second day, and so forth. However, even within the same day, the Infection Situation may change between morning and evening. Therefore, if it is desirable to improve the accuracy of Infection Countermeasures, finer units, such as one hour, can be used. Note that, even if the unit is set to one day, it is not necessary to collect continuously for 24 hours. For example, it is acceptable to collect for just one hour starting from 13:00 each day and this amount can be used for that day.

Similarly, in the following, time-varying quantities will be treated as sequences of numbers or sequences of values by discretizing time. Moreover, unless otherwise specified, if the time is not indicated in brackets [], it denotes the entire sequence. That is, in the above example, VXi denotes the entire sequence of numbers, while VXi[t] denotes the t-th value of the sequence.

<Observation Region>

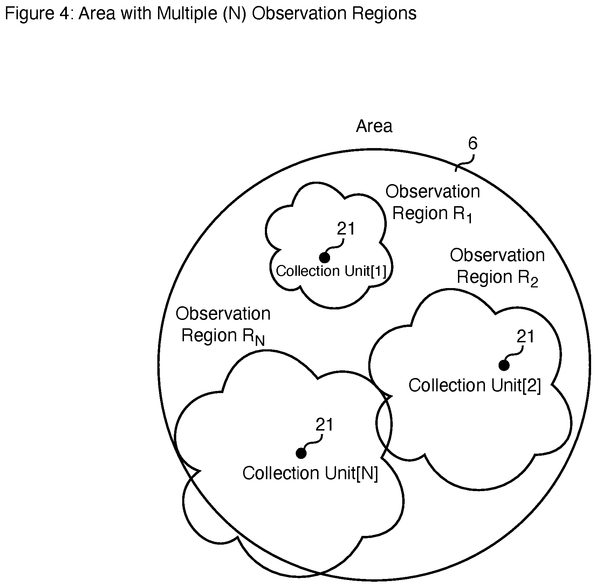

Often, a single Observation Unit 2 is insufficient to measure the amounts of Propagating Substances throughout the entire Area 6. Moreover, the Infection Situation within Area 6 may not be uniform and could vary by location. In such cases, relying solely on a single measurement result may not allow for detailed Infection Countermeasures. Therefore, generally, the Observation Unit 2 is considered to measure the Propagating Substances in a smaller space referred to as the "Observation Region" (Figure 3 and Figure 4). However, even in that case, the measurement results of the Observation Unit 2 do not necessarily accurately represent the amounts of Propagating Substances within the Observation Region. In that case, adjustments should be made until sufficient accuracy is achieved (for details, refer to <Verification Methods for Approximation Accuracy> described later) by narrowing the range of the Observation Region or by strengthening the measurements of the Observation Unit 2 (such as extending the observation time or conducting collection over a wider area within the Observation Region). For example, if an Observation Unit 2 is installed indoors within a building, including the outdoor area of the building in the Observation Region may be inappropriate, as the amounts of Propagating Substances can vary significantly between the indoor and outdoor areas. In that case, it would be better to limit the Observation Region to the said indoor area. If, as a result, the Observation Region becomes too small, measures should be taken by moving the Observation Unit 2 outdoors or adding another unit to install both indoors and outdoors. Note that it is ideal for the Observation Region to fit within Area 6. However, it may extend outside or even be completely outside of it. Additionally, Area 6 itself may also serve as the Observation Region.

<Analysis Unit 3>

The Analysis Unit 3 consists of a "Memory Unit 31," a "Decision Unit 32," and an "Update Unit 33." The Decision Unit 32 performs estimation of the Infection Situation and Infection Control using the "Decision Process." The Update Unit 33 optimizes the control of Decision Unit 32 by feeding back the results of the control using "Update Process." Note that the "Infection Quantification Function" internal to the Decision Process and Update Process is a central function of the present disclosure.

<Analysis Device 5>

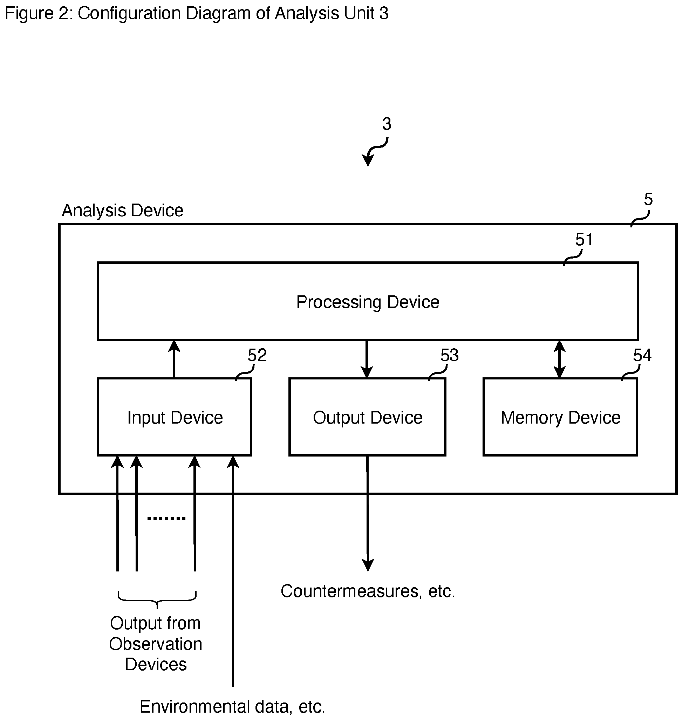

Figure 2 is a configuration diagram of the device that implements the Analysis Unit 3 (hereinafter referred to as the "Analysis Device 5"). The Analysis Device 5 consists of a "Processing Device 51" (typically implemented by a CPU, i.e., Central Processing Unit), a "Memory Device 54" (typically implemented by RAM, i.e., Random Access Memory), an "Input Device 52," and an "Output Device 53." The process of the Analysis Unit 3 (the Decision Process and the Update Process) operates by its program being stored in the Memory Device 54 and executed by the Processing Device 51. The output of the device that implements the functions of the Observation Unit 2 (hereinafter referred to as the "Observation Device") is input into the Input Device 52. The Observation Device and the Input Device 52 may be connected via a communication line or the output of the Observation Device may be manually entered into the Input Device 52 by a human. The Input Device 52 also receives input of other data, such as Environmental Data described later. Additionally, the Output Device 53 outputs information such as the "Countermeasures" described later.

<Memory Unit 31>

The Memory Unit 31 holds the "Area Information 43," "Decision Criteria 41," "Update Criteria 42," and other data described later.

<Environmental Data>

Information useful for Infection Countermeasures, other than measured values, is referred to as "Environmental Data," which is preferably provided as time series data, including not only information from the current day but also past information. Additionally, it is preferable for the data of a single day to include changes, for example, every hour. Furthermore, it is preferable to provide data not only for the entire Area 6 but also for the distribution of individual locations within Area 6.

Examples of Environmental Data include weather data (such as weather, temperature, humidity, wind direction, wind speed, sunlight hours, and precipitation within Area 6) and statistical information about the group staying in Area 6 (such as age, gender, vaccination status, antibody acquisition status, and the movement routes and duration of stay within Area 6 on that day).

<Area Information 43>

Area Information 43 is a table that records the location information of Area 6 and the location information of each Observation Region belonging to the said Area 6 (all Observation Regions if there are multiple). The location information here refers to data representing the location of Area 6 and the Observation Regions, more strictly, data that represents the boundaries enclosing those regions. A simple example would be an address. For instance, the location information of Area 6 could be "Tokyo," while the location information of an Observation Region could be "XX Town in YY City, Kanagawa Prefecture." Note that since Area 6 and the Observation Regions are generally three-dimensional regions, it is necessary to specify not only the horizontal extent but also the vertical extent. For example, it would be acceptable to define the vertical extent as "the height of the tallest building in the specified address." Additionally, it is permissible to define the three-dimensional shape of the region as polygons using coordinates (latitude, longitude, and altitude).

The table consists of multiple entries. The first entry records the location information of Area 6. The next entry records the location information of Observation Region R1. Similarly onward, the location information of Observation Regions R2 and beyond is recorded one per entry.

<Countermeasure>

The output of the Decision Unit 32 is referred to as "Countermeasures," which serves as the output of the Infection Countermeasure System 1 itself. For example, it outputs countermeasures such as "do nothing," "enhance Infection Situation monitoring through random testing," "issue stay-at-home orders," "lockdown for L days," "early termination of lockdown," and "enhance the preparation to accept infected people in hospitals." Here, L is an appropriate numerical value, which can be predetermined or calculated by the Decision Unit 32. Additionally, it is acceptable to output Countermeasures for each Pathogen Type, such as "spraying agents effective against Pathogen A."

In the following, the only Countermeasure considered will be "lockdown for L days," and the Decision Unit 32 is assumed to output L. Here, L is a positive integer or 0, where L=0 specifically means "do nothing."

<Decision Unit 32>

The Decision Unit 32 executes the Decision Process by referencing the Decision Criteria 41 and outputs the Countermeasure. The Decision Process utilizes measured values VAi, VBi, VCi, ... (where N represents the number of Collection Units 21, and i represents all integers from 1 to N).

In the present disclosure, the Decision Process is executed exactly at 24:00 midnight, and this time is referred to as the Decision Time Td. For simplicity, the process is assumed to be completed instantaneously, and in the case of a lockdown, it is to begin exactly at 0:00 a.m. at time Td + 1.

<Decision Criteria 41>

The rules for the Decision Process to calculate the Countermeasure are the "Decision Criteria 41." In the present disclosure, the Decision Criteria 41 is defined as a table. However, more generally, it may also be defined as a function that calculates the Countermeasure from measured values and Environmental Data.

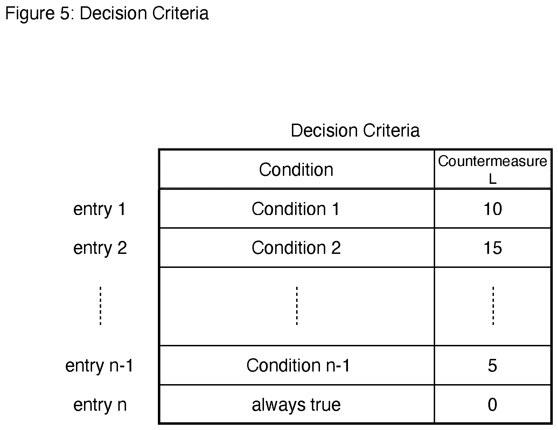

The table consists of one or more entries. Each entry is composed of a "Condition" and a "Countermeasure L" (Figure 5). The Decision Process examines the entries in the table sequentially, starting from the first entry, and outputs the "Countermeasure L" of the first entry for which the "Condition" evaluates to true.

Note that the last entry of the table can be set so that the "Condition" is always true and the "Countermeasure L" is 0 (hereinafter this entry is referred to as the "Decision Sentinel"). This ensures that some result is always output from the Decision Process, even if the "Condition" evaluates to false for all other entries.

<Update Method>

In the process performed by the Update Unit 33 (Update Process), the method of rewriting the Decision Criteria 41 is referred to as the "Update Method." Examples of this method include rewriting the "Condition" and "Countermeasure L" of entries, adding or deleting entries, but no specific constraints are imposed.

In the following, we will consider only the case of rewriting the "Countermeasure L" as the Update Method. Specifically, the Update Method is a single integer (a positive, negative value, or 0) "ΔL," meaning "to rewrite to the value obtained by adding ΔL to L. However, if the addition results in L<0, it is replaced with L=0 (i.e., L is always maintained to be L≥0)."

<Update Unit 33>

The Update Unit 33 executes the Update Process by referencing the Update Criteria 42, calculates the Update Method, and rewrites the Decision Criteria 41 accordingly. Note that the Update Unit 33 may be omitted if the Update Process is not performed.

<Update Criteria 42>

The rules for the Update Process to calculate the Update Method ΔL are the "Update Criteria 42." In the present disclosure, the Update Criteria 41 is defined as a table. However, more generally, it may also be defined as a function that calculates the Update Method ΔL from measured values and Environmental Data.

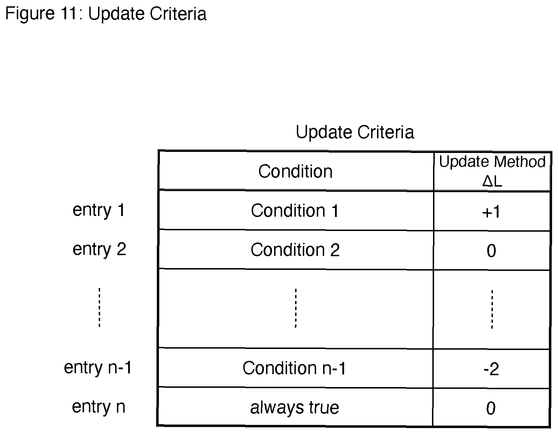

The table consists of one or more entries. Each entry is composed of an "Condition" and an "Update Method ΔL" (Figure 11). The Update Process examines the entries in the table sequentially, starting from the first entry, and updates the Decision Criteria 41 using the "Update Method ΔL" of the first entry for which the "Condition" evaluates to true.

Note that the last entry of the table can be set so that the "Condition" is always true and the "Update Method ΔL" is 0 (hereinafter this entry is referred to as the "Update Sentinel"). This ensures that some result is always output from the Update Process, even if the "Condition" evaluates to false for all other entries.

The Update Criteria 42 may be omitted if the Update Process is not performed.

Now that we have finished defining the common terminology, we will proceed to the details of the first Embodiment.

<Suspension Quantity qi, Emission Quantity ei, Residence Quantity si>

In the present Embodiment, we consider only one Collection Unit 21 (i=1 only) and pathogen A (e.g., coronavirus) as the only type of Propagating Substance. Additionally, it is preferable that the amount of A produced in nature is negligibly small and that the A present within the Observation Region Ri be considered to have been produced within the bodies of the people staying there. Note that the following discussion holds true even if a measured value VBi, which measures another propagating substance B, is used instead of the measured value VAi, if VAi and VBi are in a proportional relationship.

The number of A per cubic meter in Ri at time t (i.e., t-th day of Infection Countermeasures) is denoted as qi (hereinafter referred to as the "Suspension Quantity"). The average number of A emitted per person per day during their stay in Ri is denoted as ei (hereinafter referred to as the "Emission Quantity"). Furthermore, the cumulative duration of stay of the group within Ri (measured in person-days; for example, 10 person-days could represent 1,000 people staying for 0.01 days each, or 100 people staying for 0.1 days each; hereinafter referred to as the "Residence Quantity") is denoted as si. The cumulative duration of stay can also be referred to as the cumulative duration spent outside, if residences and offices within Ri are excluded from consideration. To measure the Residence Quantity si, for example, a stopwatch could be given to everyone staying in Ri on that day, and each person would measure their own duration of stay. The duration of stay for all individuals can then be summed.

Note that since qi, ei, and si are time-varying quantities, they should originally be written as qi[t], ei[t], and si[t]. However, since t is fixed in the present Embodiment, the notation "[t]" will be omitted unless it causes particular confusion. The values defined as "time-varying quantities" below will be treated in the same manner. Additionally, below we will often deal with quantities proportional (including approximate proportionality) to qi, ei, and si rather than themselves. These are denoted with a prime (') as qi', ei', and si', and are referred to as the "Relative Suspension Quantity," the "Relative Emission Quantity," and the "Relative Residence Quantity," respectively.

If the Collection Unit 21[i] collects A present in the air within the Observation Region Ri without bias, it can be said that VAi is proportional to qi, so VAi will be denoted as qi' hereafter. Additionally, as a first-order approximation, the larger the qi, the larger the amount of A taken into the bodies of people staying within Ri, and thus it can be said that the Infection Situation is worse. In Examples 1 to 4, it was observed that qi' is correlated with the future number of newly confirmed people in the case of the coronavirus.

Furthermore, A present within Ri exists in various forms such as droplets, aerosols, and droplet nuclei. It can be said that if qi doubles, as a first-order approximation, the amount of A in the form of, for example, droplets (similarly for aerosols and droplet nuclei) would also double. Therefore, if qi doubles, the amount of A in droplet form taken in by people staying within Ri would also double. This means that, even if A only causes droplet infection (and does not cause aerosol or airborne infection), it is not necessary to limit the collection of A to those in droplet form when measuring qi. That is, when the amount of A in the forms of droplets, aerosols, and droplet nuclei is collected and measured comprehensively without distinction, and is denoted as qi, then as a first-order approximation, qi is proportional to the amount of A in droplet form taken in by people staying within Ri.

Strictly speaking, however, as discussed below, it cannot necessarily be said that the larger the qi, the worse the Infection Situation. And when implementing Infection Countermeasures with greater accuracy than a first-order approximation, various factors should be considered: First, even with the same qi value, the assessment of the Infection Situation may vary depending on whether the number of people with antibodies is large or small. Second, depending on the pathogen, taking it into the body may have both positive and negative effects (e.g., activating the immune system, which may make it less likely to contract other diseases). Third, the collected A was emitted through various actions such as sneezing, talking, and breathing by infected people. It then floated in the air before being eventually captured by the Collection Unit 21. Even if emitted as droplets, some may become droplet nuclei before being collected, while others may remain as droplets, so the form is not uniform. Additionally, the proportion of these may vary depending on whether the air is dry on that day. Fourth, depending on the condition of the infected person (for example, whether in the early stages of infection or later), the proportion of A emitted as droplets, aerosols, or droplet nuclei may vary (for example, in the early stages of infection, the ratio for each might be 8:2:1, but later it could change to 2:1:1). Additionally, other factors may also come into play.

Next, considering the perspective of a person emitting the pathogen, as opposed to one taking it in, it can be said that, as a first-order approximation, the larger the ei, the larger the amount of pathogen in the body and the worse the Infection Situation.

Strictly speaking, however, as discussed below, it cannot necessarily be said that the larger the ei, the worse the Infection Situation. And when implementing Infection Countermeasures with greater accuracy than a first-order approximation, various factors should be considered: First, even with the same ei value, the assessment of the Infection Situation may vary between better and worse depending on the breakdown-such as whether there are many mild cases (or cases with a small amount of pathogen emitted) or a few severe cases (or cases with a large amount of pathogen emitted). In particular, among the former mild cases, there would be those whose symptoms are so mild that it is hard to consider an infection, recovering within a few hours without any subjective symptoms. (According to the inventor's experience, if the amount of inhaled coronavirus is small, the discomfort in the throat subsides within tens of seconds, but if the amount is large, it may take several hours to subside. During this subsidence, no specific symptoms appear, but the inventor themselves is likely emitting a trace amount of the virus. Others would be in a similar situation, just without realizing it.) In such cases, the proportion of mild and severe cases would be separately determined or estimated to assess the Infection Situation. Second, depending on the type of pathogen, the amount emitted may peak before the onset of symptoms, so a large amount of pathogen emitted by a person does not necessarily correspond with the severity of their illness. Third, the relationship between the amount of pathogen emitted by an infected person and the amount of pathogen in their body may be more complex than a proportional one (such as in diseases other than those within the respiratory system). Fourth, even with the same $e_i$ value, if the nature of the pathogen changes due to mutation, the assessment of the Infection Situation may vary. Additionally, other factors may be involved.

Here, we consider the relationship between $q_i$ and $e_i$. The total amount of A emitted within $R_i$ on that day can be calculated as $e_i*s_i$. As a first-order approximation, if this total amount doubles, $q_i$ will also double. Therefore, $q_i$ and $e_i*s_i$, or equivalently, $q_i/s_i$ and $e_i$, are in a proportional relationship. Strictly speaking, however, $q_i$ may also include A that has entered from outside the Observation Region $R_i$. Nevertheless, if the Infection Situation and the amount of people near the boundary surface of $R_i$ do not change significantly, the amount of A entering from the outside through a certain portion of the boundary surface is considered to be balanced and offset by the amount of A leaving through the same portion. Therefore, when defining the Observation Region $R_i$, if the boundary surface is set in this way, it is acceptable for the amount of A entering from the outside to be included in $q_i$, as a first-order approximation.

Note that, instead of determining $s_i$, it is also acceptable to determine a value $s_i'$ proportional to it. For example, let $s_i'$ denote the cumulative duration of stay in a portion of $R_i$ (such as a square area 100 meters on each side at the center of $R_i$), instead of the entire $R_i$. As a first-order approximation, if $s_i$ doubles, $s_i'$ also doubles. Therefore, $s_i$ and $s_i'$ are in a proportional relationship. Furthermore, if we define $e_i'=q_i'/s_i'$ (hereafter referred to as "Equation EQS"), then $e_i'$ is proportional to $e_i$.

In this way, to obtain quantities such as $q_i$, $e_i$, and $s_i$, it would often be the case that the quantities with a prime, such as $q_i'$, $e_i'$, and $s_i'$, are measured first. Furthermore, it would often be the case that Infection Countermeasures are implemented using only the quantities with a prime. For example, $s_i'$ may be measured while $s_i$ is not determined.

<Verification Methods for Approximation Accuracy: $q_i$ and $q_i'$, $e_i$ and $e_i'$, $s_i$ and $s_i'$>

The proportional relationships between $q_i$ and $q_i'$, $e_i$ and $e_i'$, and $s_i$ and $s_i'$ mentioned above are first-order approximations. Whether the approximation accuracy is sufficient can be verified by conducting the following procedure over multiple days (with the number of days determined based on the required accuracy). First, for $s_i'$, while the previous example considered a region 100 meters on each side, the cumulative duration of stay in a wider region (the closer to $R_i$, the better, but deter-

mined based on the required accuracy) should be examined to check if it is in a proportional relationship with $s_i'$. Next, for $q_i'$, A should be collected while moving within $R_i$ without bias over a longer period of time (the duration and distance are determined based on the required accuracy), and the amount should then be measured to check if it is in a proportional relationship with $q_i'$. Finally, for $e_i'$, multiple people (the number of people is determined based on the required accuracy) who stayed in $R_i$ on the day should be selected randomly, and the average amount of A they emit should be measured to check if it is in a proportional relationship with $e_i'$. If a proportional relationship holds, the accuracy of $q_i'$ and $s_i'$, which were used to calculate $e_i'$, can be indirectly inferred to be sufficient. Note that, in this case, for example, if the amount of A emitted as droplets represents the severity of the Infection Situation, it would be sufficient to compare the average amount emitted specifically as droplets (excluding aerosols and others) with $q_i'$. Furthermore, if any of the above procedures yield insufficient accuracy, it can be addressed by changing the measurement methods for $q_i'$, $e_i'$, and $s_i'$, altering the installation location of the Collection Unit 21[i], modifying the range of $R_i$, and so on.

Additionally, whether the relationship discussed previously-namely, the higher the $q_i$ or $e_i$ (or $q_i'$ or $e_i'$), the worse the Infection Situation-holds can also be verified using the above method of random sampling to assess health condition. That is, the relationship between the amount of $q_i$ or $e_i$ and the health condition of randomly selected people (including not only their condition on that day, but also, if necessary, their condition on subsequent days reflecting, for example, the result of the pathogen taken into their bodies on that day) is examined. If it holds true that the larger the $q_i$ or $e_i$, the worse the Infection Situation, then the magnitude of $q_i$ or $e_i$ can be directly regarded as the severity of the Infection Situation. Even if that is not the case-namely, if the relationship between $q_i$ or $e_i$ and the Infection Situation is more complex, such as nonlinear-measures can still be taken. For example, if there is a threshold such that "if $q_i$ or $e_i$ exceeds that threshold, the Infection Situation is definitely worse," Infection Control can be implemented when $q_i$ or $e_i$ exceeds that threshold.

In the previous discussions, the Infection Situation was assessed based on the quantity of either $q_i$ or $e_i$. However, what should be done in cases where, for example, $q_i$ is large but $e_i$ is small, or vice versa? Which indicator is more important may vary depending on the objectives, content, and other circumstances of the Infection Countermeasures. For example, in the case of Infection Countermeasures that issue stay-at-home advisories for people with compromised immunity, it may be more appropriate to focus on the magnitude of $q_i$ rather than $e_i$. This is because, even if $e_i$ is small, if $q_i$ increases, the number of locations with infection risk (as a first-order approximation) increases, thereby increasing the probability of infection through going out. On the other hand, if, for example, the extent of infection spread is important, then even if qi is small but ei is large (for instance, the infection is spreading but the amount of going out on that day happens to be extremely small), a lockdown may be necessary. Additionally, as in Examples 1 to 4, where qi is correlated with the number of newly confirmed people, the response may vary depending on the magnitude of ei (the extent of infection spread), even for the same value of qi. This is because, for example, even if the number of newly confirmed people on that day is the same at 100, the severity differs depending on whether there are already 10,000 total infected people or only 10. That is, there may be cases where both qi and ei are considered when implementing Infection Countermeasures. Moreover, when implementing Infection Countermeasures with greater accuracy than a first-order approximation, various factors should be considered in addition to qi and ei.

<Examples of Infection Countermeasures>

Once the relationship between the Relative Suspension Quantity qi', Relative Emission Quantity ei', and the Infection Situation is understood through methods such as those described above, Infection Countermeasures can be implemented using qi' and ei'. However, as discussed so far, this relationship can be simple, such as a proportional relationship, or more complex. Additionally, while there are various methods for Infection Countermeasures, the main focus of the present disclosure is not to indicate which method is preferable. Therefore, in the following, a general framework will be presented, and, as an example, a simplified method will be explained. In this method, the Infection Situation is considered to have worsened when qi" or ei" exceeds a certain threshold (referred to as the "Suspension Threshold" Qi" and the "Emission Threshold" Ei", respectively), and lockdowns are implemented as a measure of Infection Control. However, it is not necessarily required to use both qi' and ei', and Infection Countermeasures can be implemented using either qi' or ei' alone. Naturally, in such cases, there is no need to determine unnecessary values.

Note that it is desirable to update the thresholds Qi' and Ei' as needed to maintain accuracy, even after they have been initially set. This is because the properties of the pathogen, the group's resistance to the pathogen, the climate, and other factors may change over time, which can alter the relationship between ei', qi', and the Infection Situation. Additionally, the values of Qi' and Ei' may be changed according to environmental conditions such as the temperature and humidity on that day. For example, in cases with temperatures and humidity suitable for the expansion of infection, it may be appropriate to use a lower value for Qi'.

Furthermore, the scope of Infection Control does not necessarily have to align with Ri. It may be only part of Ri, a region that encompasses Ri such as the entire Area 6, or a region that does not overlap with Ri but is considered to have a similar Infection Situation as Ri, among others. For example, in the case of a lockdown, various controls can be considered, such as "prohibition of going out for people who stayed in Ri on that day," "prohibition of going out for everyone who might have stayed in Ri on that day," "prohibition of entry into Ri," "prohibition of entry into regions with a similar Infection Situation as Ri," or "prohibition of going out for the entire Area 6."

<Decision Process 1>

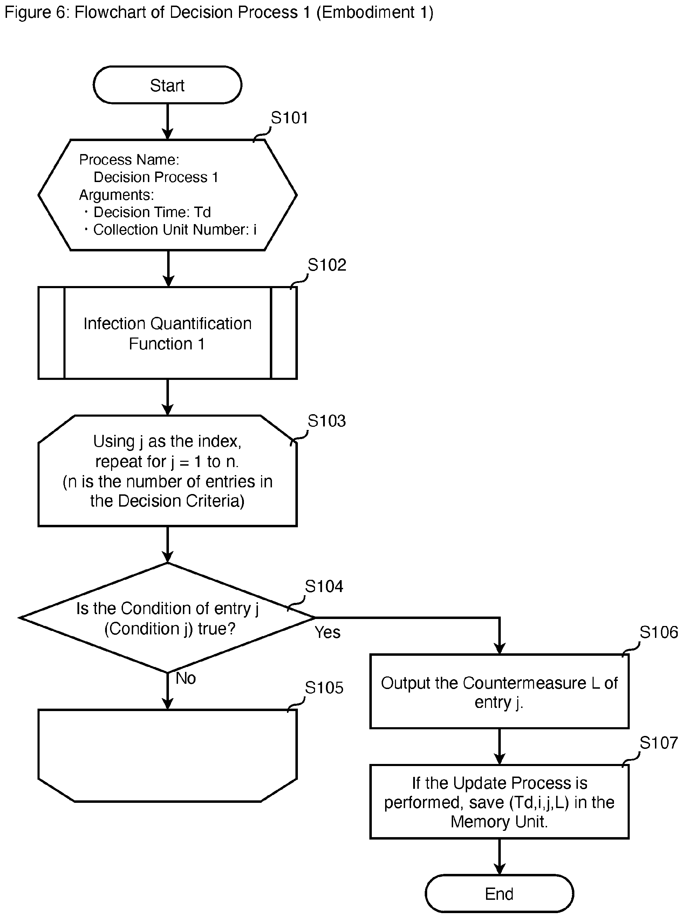

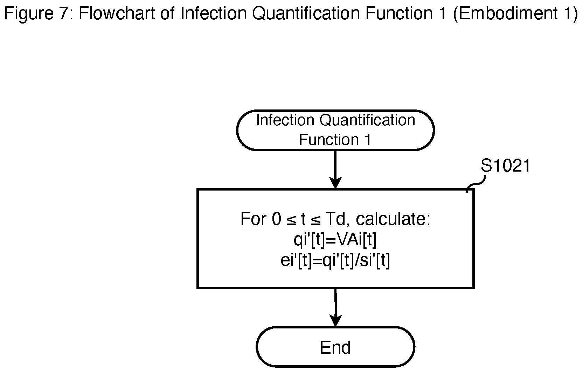

The Decision Process in the present Embodiment will be explained using the flowchart in Figure 6 (referred to as the "Decision Process 1", but simply called "Decision Process" in this context). This Decision Process calls the Infection Quantification Function 1 in Figure 7 as a subroutine. Note that the following symbols will be used below: measured value VAi, Relative Suspension Quantity qi', Relative Emission Quantity ei', Relative Residence Quantity si', Suspension Threshold Qi', and Emission Threshold Ei'. To ensure accuracy, brackets [] will be included without omission.

The Decision Process starts with the arguments of Decision Time: Td and Collection Unit Number: i (S101). Next, the Infection Quantification Function 1 is called (S102). Where, for times t satisfying $0 \leq t \leq Td$, qi'[t] =VAi[t] and ei'[t]=qi'[t]/si'[t] are calculated (S1021). Note that, as mentioned repeatedly, values not referenced by the Decision Criteria 41 may be omitted from the calculation here (similarly, in subsequent Embodiments, values not referenced may be omitted from the calculation in the Infection Quantification Function). Next, using j as an index, repeat the following from the first entry (j=1) of the Decision Criteria 41 to the last entry (j=n, with n as the number of entries in the Decision Criteria 41) (S103). First, check whether the Condition of entry j (Condition j) is true. At this point, it is permissible to refer to Td, i, qi', ei', si', Qi', Ei', and Environmental Data from the Condition as needed (S104). If Condition j is true, the Countermeasure L for that entry is output (S106). Then, if the Update Process described in a later Embodiment is to be performed, save (Td, i, j, L) for that case (S107). On the other hand, if Condition j is false, return to the beginning of the loop (S105).

For example, assume that the Decision Criteria 41 consists of the following 3 entries: (1) Condition="-qi'[Td]$\geq$Qi'*1.5", Countermeasure L=20. (2) Condition="-qi'[Td]$\geq$Qi'", Countermeasure L=10. (3) Decision Sentinel.

In this case, the Decision Process will output "L=0" if qi'[Td]<Qi', as the Infection Situation is considered good. Additionally, if Qi'$\leq$qi'[Td]<Qi'*1.5, it will output "L=10", as the Infection Situation is considered poor. Furthermore, if qi'[Td]$\geq$Qi'*1.5, it will output "L=20", as the Infection Situation is considered very poor.

In the example of the Decision Criteria 41 mentioned above, only qi' and Qi' are referenced in the "Condition" of each entry, but as mentioned earlier, any of the values qi', ei', Qi', or Ei' may be freely used. Furthermore, in the "Countermeasure" part as well, the value of L can be determined using the values of qi', ei', Qi', or Ei', with, for example, "L=(ei'[Td]/Ei')*10" (note that this formula is merely an illustrative example). This approach allows the lockdown duration to be changed based on the magnitude of ei', even with the same value of qi'. In other words, even if the value of qi' on that day is the same, there are cases with small ei' and large si', or with large ei' and small si'. In the latter, the lockdown can be implemented for a longer duration by considering the infection spreading more than the former.

When the output of the Decision Process is L>0, the lockdown is initiated, and typically, there is no need to execute the Decision Process again until the lockdown ends after L days. However, it is also possible to use the Decision Process to determine an early termination to the lockdown. In other words, even during the lockdown, it is permissible to execute the Decision Process again at times t (where Td<t<Td+L) as appropriate to monitor qi' and ei'. (However, the reliability of qi' and ei' values may be lower during the lockdown due to reduced amount of going out. In this case, it is also permissible to measure qi' and ei' after temporarily increasing the amount of going out by allowing only part of the group to go outside on that day, for example.) One example of time t could be the midpoint of the lockdown, i.e., t=L/2 (with any decimal points in L/2 rounded up). In this case, for example, if the output Countermeasure L is L=0, the lockdown can be terminated early, considering the Infection Situation has improved. If L>0, the lockdown will continue.

<Summary of the Present Embodiment>

In the present Embodiment, Infection Countermeasures were implemented using the Relative Suspension Quantity qi' and the Relative Emission Quantity ei'. Infection Countermeasures can most easily be implemented by considering that the Infection Situation within the observation region Ri is in a proportional relationship with qi' or ei', but they may also have a more complex relationship.

Furthermore, in Examples 1 to 4, we observed that qi' measured in the Observation Region Ri, which is several kilometers in size in Zone A, was correlated to the future number of newly confirmed people in the large Area 6, Tokyo. In other words, by measuring qi', it is possible to predict the increase, decrease, and peaks of the Infection Situation within a large Area 6 at a practical cost and implement Infection Control at an earlier stage. Meanwhile, currently in Japan, the increase, decrease, and peaks of the Infection Situation are determined by looking at the statistics of the number of newly confirmed people. Similarly, by determining ei', it is possible to estimate the Infection Situation within a large Area 6 on the same day at a practical cost and implement Infection Control.

Although the method of detecting viruses in the air to determine if there are infected people nearby may occur to someone, they typically don't try to implement it. This is because, based on experience, it is believed that such a method can provide information only about the Infection Situation in the immediate vicinity (e.g., within a square area with a side length of a few tens of meters), and assessing the Infection Situation in larger areas like the entire Tokyo would require significant costs. This is because, with such a method, at best, only the infection situation in a nearby area (for example, a square area with sides of several tens of meters) can be understood, and it would require an enormous cost to grasp the Infection Situation across a large area, such as the entire Tokyo, based on experiential understanding. However, that is not the case. The important point of the present disclosure is that, in fact, it can be done at a very low cost.

Furthermore, as described in Example 1, even in the early stages of infection spread, it is expected that by merely measuring the amount of virus in the air, Infection Situations can be estimated and Infection Control can be implemented early and at low cost, even if statistical data on newly confirmed people is not sufficiently available.

[Embodiment 2]

In the present Embodiment as well, we consider one Collection Unit 21 (i=1 only) and Pathogen Type A as the only type of Propagating Substance. Additionally, it is preferable to assume that A is also absent in environments where no infected people are present. Note that the following discussion also holds if a measured value VBi of another Propagating Substance B is used instead of the measured value VAi, provided that VAi and VBi are in a proportional relationship. Furthermore, in the present Embodiment as well, Infection Countermeasures at a specific time t are considered.

<Absorption Quantity ai>

In the present Embodiment, Infection Countermeasures using a quantity referred to as "Absorption Quantity" are explained. Define the Absorption Quantity ai as the product of the Suspension Quantity qi and the Residence Quantity si within the Observation Region Ri at a given time t. That is, ai=qi*si, which is a time-varying quantity. In addition, quantities proportional to ai (including approximate proportionality) are denoted with a prime as ai', and referred to as the "Relative Absorption Quantity."

As seen in Embodiment 1, qi is proportional to the amount of A that an individual staying in Ri takes into their body over a certain period of time. Therefore, ai is proportional to the total amount of A taken in by all people staying within Ri during their stay. As a first-order approximation, it can be said that the larger the ai, the worse or more deteriorated the Infection Situation of the group (i.e., all people staying in Ri). This is because such an amount of pathogens has entered the bodies of the group and gained the opportunity to proliferate. Some people might become newly infected due to that, and even for those already infected, it is better to take in a smaller amount of pathogens.

However, when implementing Infection Countermeasures with greater accuracy than a first-order approximation, various factors should be considered as in the discussion in Embodiment 1 (the relationship between qi, ei, and the Infection Situation). For example, even with the same value of ai, the assessment of the Infection Situation may change depending on the number of people with antibodies.

The relationship between the Infection Situation of a group and ai can also be specifically illustrated as follows. Assume for the moment that the probability of infection is represented by a function f(x), where x is the number of pathogen A units taken into the body (for example, there are studies that model this with $f(x)=1-\exp(-x/N)$, using N as a parameter: https://www.medrxiv.org/content/10.1101/2020.10.21.20216895v1.full). When plotting the graph of the function f(x), if there exists a tangent to f(x) at x=0 and its slope is k, then as a first-order approximation, the proportional relationship $f(x)=k*x$ (hereafter referred to as Equation FKX) holds.

Furthermore, as for the quantity of the group si, let us assume that its breakdown is <n1,d1>, <n2,d2>, <n3,d3>, for example. Here, <n1,d1> denotes that there were n1 people who stayed in Ri for d1 days. Since the amount of A taken in per person for these n1 people is proportional to d1*qi, if f(x) and x are in a proportional relationship, then the number of people infected will be proportional to n1*d1*qi. The same applies to n2 and n3, so the total number of people infected in Ri on that day is proportional to n1*d1*qi+n2*d2*qi+n3*d3*qi. By transforming this equation with attention to si=n1*d1+n2*d2+n3*d3, we find that it is equal to si*qi, namely ai. However, this is a first-order approximation, and since the group represented by n1+n2+n3 includes people who are already infected, it would be more accurate to exclude them from the count. The above discussion holds for other breakdowns of si as well, so ultimately, if the number of newly infected people in Ri on that day is represented as pi, then as a first-order approximation, pi is proportional to ai.

In general, there is an incubation period when a person is infected with a pathogen. In other words, the infection is often discovered after some time has passed, when the infection becomes apparent. In contrast, if pi can be estimated, Infection Control for the group can be implemented at an earlier stage.

In the above, the number of newly infected people was determined, but more generally, the "number of people for whom the probability f(x) of reaching that condition is proportional to x" can also be determined. For example, if the probability of sneezing three or more times due to taking in A while staying within Ri is f(x), and f(x) is proportional to x, then the number of people who sneeze three or more times due to A within Ri on that day is also proportional to ai. Additionally, in general, when a certain quantity used in Infection Countermeasures is proportional (including approximate proportionality) to ai, it will be referred to as the "Influence Quantity". Using ai, various Influence Quantities can be estimated.

In the present Embodiment, Infection Countermeasures are implemented using a quantity ai' that is proportional to ai. First, as in Embodiment 1, the measured value VAi is used as the Relative Suspension Quantity qi' that is proportional to qi. Similarly to the Relative Residence Quantity si' in Embodiment 1, using a quantity si" that is proportional to si but has two primes (hereinafter referred to as the Relative Residence Quantity as well), we define ai'=qi'*si" (hereinafter referred to as "Equation AQS"). The reason for using two Relative Residence Quantities, si' and si", here is that they do not need to have the same value, and they can also have different levels of accuracy. In other words, the Relative Residence Quantities used to determine ei' from qi' using Equation EQS (ei'=qi'/si') and those used to determine ai' from qi' using Equation AQS can be different. Naturally, it is also acceptable to use the same value by setting si"=si'.

Additionally, in Embodiment 1, ei' was determined from qi' and si' using Equation EQS, but in the present Embodiment, ei' may be determined through actual measurements (such as the random sampling method described in Embodiment 1). Then, ai' can be determined from ei', si', and si" using the equation ai'=ei'*si'*si" (hereinafter referred to as Equation AESS). Note that, in this case, if the two values si' and si" do not need to be determined separately and their product (si'*si") can be measured directly, it can be multiplied with ei'.

Below, we will explain the case of estimating the number of newly infected people, pi, which is a representative Influence Quantity, to implement Infection Countermeasures. Note that this method can also be applied to other Influence Quantities.

If the cumulative duration of stay that is limited to people who are not infected at time t (obtained from the Relative Residence Quantity si' by subtracting the cumulative duration of stay of people who are already infected on that day) is measured or estimated as the Relative Residence Quantity si", the estimation of the number of newly infected people pi from the previous discussion will be more accurate. This is a similar discussion to when determining the relationship between ai and the Infection Situation with greater accuracy than a first-order approximation.

By the way, in Examples 1 to 4, we observed that qi'=ei'*si' is correlated with pi. If the proportional relationship between pi and ai mentioned above holds, then in practice, ai'=qi'*si"=ei'*si'*si" is proportional to pi. Comparing qi' with the definition of ai', it can be said that qi' is an approximate value of ai' by setting si" = 1. Alternatively, it can be said that qi' is an approximate value of ai' obtained by replacing each of si' and si" with the square root of si' $(\sqrt{si'})$. Therefore, if the Residence Quantity (si or si") is constant, qi' is proportional to ai', and the accuracy of the approximation of qi' to pi can be considered high. Otherwise, if the Residence Quantity multiplies by 1.5, for example, ai' will multiply by 2.25, whereas qi' will multiply only by 1.5, resulting in an error in the approximation of pi by qi'.

<Verification Methods for Approximation Accuracy: ai and ai', pi and ai>

The proportional relationships between ai and ai', and between pi and ai, defined so far are first-order approximations. We will explain a method for verifying the adequacy of the approximation accuracy using the relationship between ai' and pi as an example. If the accuracy of the proportional relationship between ai' and pi is sufficient, it can be inferred, though indirectly, that the accuracy of the proportional relationships between ai' and ai, and between pi and ai, is also sufficient.

First, select multiple people (denoted as X people) randomly from either those who entered the Observation Region Ri from the outside on the given day (referred to as the stay day) or those staying in Ri exactly at 00:00 on the stay day, and isolate them. The timing for isolation is as follows: for the former case, at the moment of entry (or the first moment of entry for people who are expected to enter and exit Ri multiple times), and for the latter case, exactly at 00:00. Next, select multiple people (denoted as Y people) randomly from either those who left from within Ri on the stay day or those who were in Ri exactly at 24:00 on the stay day (excluding anyone who exited and re-entered Ri at least once on the stay day), and isolate them. The timing for isolation is as follows: for the former case, at the moment of leaving Ri (or the first moment of leaving for people who are expected to enter and exit Ri multiple times), and for the latter case, exactly at 24:00. Then, isolate those X+Y people for a sufficient period and repeatedly test them (such as measuring the amount of pathogens in their bodies using PCR tests) to examine whether they are infected. Also, let N denote the total number of people who stayed in Ri on the stay day. The numbers X and Y are determined based on the required accuracy.

As a result, let X' among X people and Y' among Y people be confirmed as infected. The infections among these X'+Y' people are either due to staying in Ri on the stay day (referred to as the day-of origin) or due to a cause prior to that (referred to as the pre-existing origin). In the following, let x=X'/X and y=Y'/Y. First, since Y is selected randomly, it can be estimated that N*y among N people are infected (the total number of people from the day-of origin and the pre-existing origin). Next, since X is also selected randomly, it can be estimated that N*x among N people are infected due to the pre-existing origin. Defining the difference between these as pi=N*(y-x), the number of people infected due to the day-of origin can be estimated as pi.

By conducting the above actual measurements over multiple days (the number of days should be determined based on the required accuracy), it is possible to verify whether the proportionality between ai' and pi is sufficiently accurate. If the accuracy is not sufficient, it can be addressed by modifying the methods for measuring qi', ei', si', and si", changing the installation location of Collection Unit 21[i], altering the range of Ri, and so on. Additionally, through these actual measurements, information can be obtained on methods for estimating pi from ai' using approximations higher than first-order (i.e., second-order, third-order, etc.), allowing for improvements in Infection Countermeasures (specifically, improving the Decision Criteria 41).

<Examples of Infection Countermeasures>

Once the relationship between the Relative Absorption Quantity ai' and the Infection Situation is understood through methods such as those described above, Infection Countermeasures can be implemented using ai'. However, for the same reasons as in Embodiment 1, a general framework is presented, and, as an example, a simplified method is explained. In this method, the Infection Situation is considered to have worsened when the Relative Absorption Quantity ai' exceeds a threshold (referred to as the "Absorption Threshold" Ai'), and lockdowns are implemented as a measure of Infection Control. Naturally, it is also acceptable to implement Infection Countermeasures together with qi' and ei'.

Note that, as with Embodiment 1, even after the threshold Ai' was determined, it is desirable to update it as needed to maintain accuracy. Alternatively, the value of Ai' may be changed according to the environmental conditions of the day. Additionally, the target range for Infection Control does not necessarily have to be Ri.

<Decision Process 2>

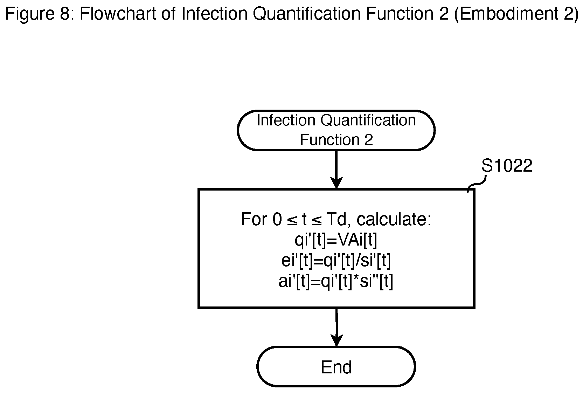

The Decision Process in the present Embodiment (referred to as "Decision Process 2," though it will simply be referred to as the Decision Process here) will be explained using Figure 6 and Figure 8. This Decision Process is the same as the Decision Process 1 in Figure 6, except that in Step S102, the Infection Quantification Function 2 from Figure 8 is called as a subroutine. The subsequent processes remain identical. Note that the following symbols will be used below: measured value VAi, Relative Suspension Quantity qi', Relative Emission Quantity ei', Relative Residence Quantities si' and si", Relative Absorption Quantity ai', Emission Threshold Ei', Suspension Threshold Qi', and Absorption Threshold Ai'. To ensure accuracy, brackets [] will be included without omission.

In the Infection Quantification Function 2, for the time t satisfying $0 \leq t \leq Td$, ai'[t]=qi'[t]*si"[t] is newly calculated (S1022). The other processes are the same as in the Infection Quantification Function 1. With this modification, si", ai', and Ai' may also be referenced as appropriate in the "Conditions" of Decision Criteria 41, in addition to Td, i, qi', ei', si', Qi', Ei', and Environmental Data. Note that Qi' and Ei' are assumed to be defined separately using methods such as those explained in Embodiment 1.

Additionally, from the Decision Criteria 41 with 3 entries illustrated in Embodiment 1, the example of Decision Criteria 41 for the present Embodiment can be obtained by replacing qi' with ai' and Qi' with Ai'.

<Summary of the Present Embodiment>

In the present Embodiment, Infection Countermeasures were implemented using the Relative Absorption Quantity ai'. Although it is simplest to implement Infection Countermeasures assuming that the Infection Situation in the Observation Region Ri is proportional to ai', more complex relationships are also acceptable.

Additionally, in Equation AQS, the relative emission quantity qi' is used to determine ai'. Therefore, similarly to the summary discussion in Embodiment 1, by using the qi' measured in a narrow Observation Region Ri, Infection Countermeasures can be implemented over a wider range (details will be described in the summary of Embodiment 3, such as the entire Area 6) at a practical cost.

And to reiterate, when ai' is proportional to the number of newly infected people pi, the number of newly confirmed people in the future can be predicted based on ai'. In addition to predictions, it is also possible to estimate the number of newly infected people in the morning of a given day. If this number is high, Infection Control can be implemented on the same day by, for example, restricting the amount of going out in the afternoon, which can reduce the number of newly infected people. The amount of pathogens in the air can significantly change from one day to the next, so it is expected to be more effective than imposing restrictions on going out the following day. In addition, being able to implement Infection Control on the same day makes it clearer what specific actions should be taken compared to implementing Infection Control after the number of newly confirmed people is known on a later day. This has the advantage of people being more actively cooperating in Infection Control.

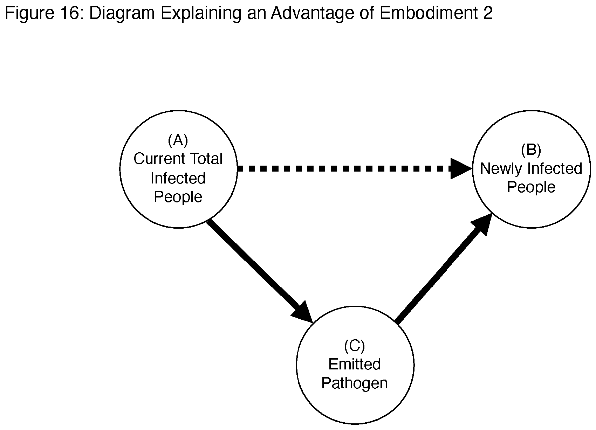

Furthermore, the advantages of the present Embodiment will be explained using Figure 16. (A) represents the total number of current infected people, while (B) represents the newly infected people. Naturally, (B) occurs as a result of viruses being transferred from (A). Therefore, as a method to estimate the number of (B) using the number of (A), concepts such as the effective reproduction number are available. On the other hand, in the present Embodiment, we consider (C) as the pathogens collectively emitted by (A). Thus, the causal relationship is not that (B) arises directly from (A) (as indicated by the dashed arrow in the figure), but rather that (A) emits (C), which in turn causes (B) to arise (as indicated by the solid arrow in the figure). Therefore, the method in the present Embodiment that estimates the number of (B) from the amount of (C) is closer in terms of causal relationship compared to the method that estimates the number of (B) from the number of (A), making estimates with higher accuracy possible. Or, at least, by improving the approximation from first-order to second-order, third-order, and so forth, there is potential to achieve such a level of accuracy.

[Embodiment 3]

In the present Embodiment, we extend Embodiment 1 by installing multiple (denoted as N, where N≥1 to include a single unit as a special case) Collection Units 21 and consider a method to improve the accuracy of Infection Countermeasures. Note that for Collection Units [i] and [j], the Observation Regions Ri and Rj may overlap each other. Additionally, we consider Pathogen Type A as the only type of Propagating Substance. It is preferable to assume that A is also absent in environments where no infected people are present.

The Infection Situation within Ri and Rj may be similar to each other in some cases and different in others. In cases when, for example, there is little movement of people or objects that mediate infection between Ri and Rj, it may be better to consider the Infection Situations in the two regions as independent and also implement the Infection Countermeasures independently. However, this would correspond to dividing Area 6 for Infection Countermeasures. So, hereafter, we assume that the N Observation Regions have similar Infection Situations to each other. In this case, the Emission Quantities ei and ej will have similar values. Moreover, as a result, if the number of people in Ri and Rj is similar, the Suspension Quantities qi and qj will also have similar values.

During the novel coronavirus outbreak that began in early 2020 in Japan, the fluctuations in the number of newly confirmed people, for example in Tokyo and Kanagawa Prefecture, have shown very similar trends. Therefore, the Infection Situations in the busy areas of these central locations (such as Tokyo Station and Yokohama Station) are considered to be similar to each other.

<Average Relative Emission Quantity e0">

In Embodiment 1, we determined the Relative Emission Quantity ei' proportional to the Emission Quantity ei, when there is only one Collection Unit 21. In the present Embodiment, as we have multiple Collection Units 21, N (1≤i≤N) values of ei' are determined. They are integrated to implement Infection Countermeasures for the entire Area 6. By doing so, more accurate Infection Countermeasures can be implemented using a broader range of information compared to when there is only one Collection Unit 21.

However, in general, since the proportionality coefficient between ei and ei' differs depending on the Collection Unit 21, simply averaging the N values of ei' would be meaningless. Therefore, ei' is first converted into a mutually comparable quantity ei" (hereinafter referred to as "Relative Emission Quantity" as well).

A representative of such a quantity is the Emission Quantity ei itself. Therefore, for example, a method can be considered in which after setting ei"=ei, their average value e0" (hereinafter referred to as the "Average Relative Emission Quantity") is determined as e0"= (e1"+e2"+ ... +eN")/N, and Infection Control for the entire Area 6 is implemented when e0" exceeds a threshold E0" (hereinafter referred to as the "Average Emission Threshold"). Alternatively, weights wi are assigned to each ei", and their weighted average is determined as e0"=(w1e1"+w2e2"+ ... +wN*eN")/(w1+w2+... +wN). The way of assigning weights, for example, gives smaller weights to less significant ei", such as ei" obtained from less accurate measured values VAi (e.g., when the performance of the Collection Unit 21 or the Measurement Unit 22 is poor) or ei" obtained from Collection Units 21 installed in less busy areas. In addition, there may be Collection Units 21 with a weight of 0. For example, if w2=0, e0" will be determined without using the measurement result from Collection Unit 21[i] (i=2). Conversely, if wi other than i=2 is set to 0, e0" will be determined using only the measurement result from Collection Unit 21[i] (i=2). It should be noted that the normal average is a special case (all weights are 1) of the weighted average.

In the above example, ei was used as ei", but generally, determining ei takes time and effort. Another example of ei" is to define ei" as a relative quantity, where the value of ei' at a certain time (for example, t=0) is set as the reference value of 1. That is, let ei"[t]=ei'[t]/ei'[0] (Note that brackets [] are written without omission here). In this case, the Emission Threshold Ei' defined in Embodiment 1 can also be converted to the corresponding threshold, say Ei", as Ei"=Ei'/ei'[0]. Furthermore, E0" is defined as the weighted average of Ei".

<Examples of Infection Countermeasures>

An example of Infection Countermeasures can be obtained from that in Embodiment 1, by replacing the Relative Emission Quantity ei' with the Average Relative Emission Quantity e0", and the Emission Threshold Ei' with the Average Emission Threshold E0". The details are described below.

<Decision Process 3>

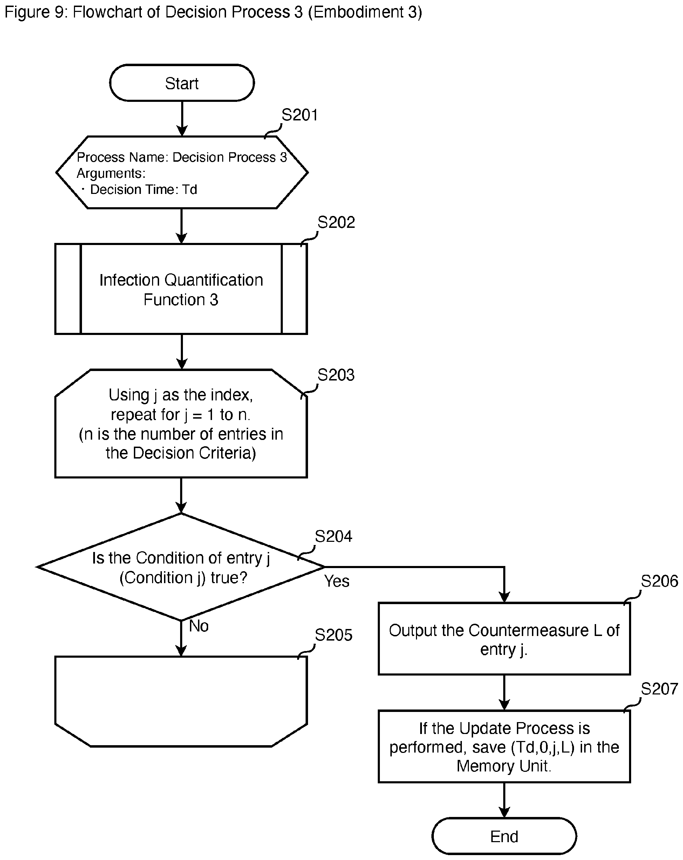

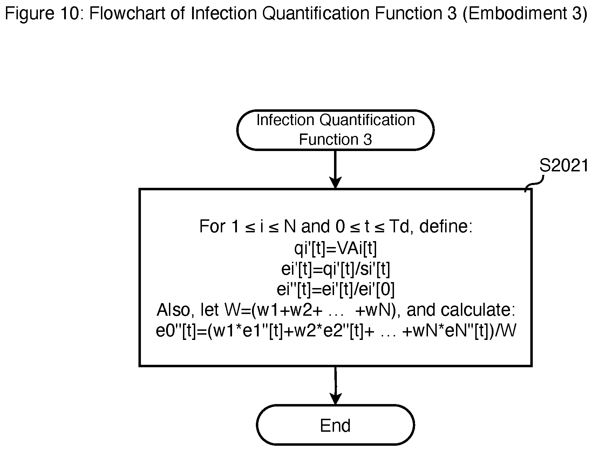

The decision process in the present Embodiment (referred to as "Decision Process 3," though it will simply be referred to as Decision Process here) will be explained using the flowcharts in Figure 9 and Figure 10.

These are similar to Figure 6 and Figure 7 in Embodiment 1, so the differences from those will mainly be explained. Note that the following symbols will be used below: measured value VAi, Relative Emission Quantities ei' and ei", Relative Residence Quantity si', weights wi, Average Relative Emission Quantity e0", Average Emission Threshold E0", and Emission Threshold Ei'. To ensure accuracy, brackets [] will be included without omission.

The difference from Figure 6 is that the parts corresponding to S101, S102, and S107 have changed to S201, S202, and S207, respectively. First, since the Decision Process in the present Embodiment integrates the measurement results from N Collection Units 21 rather than from a specific Collection Unit 21[i], S201 does not have the Collection Unit Number: i in arguments, unlike S101. For the same reason, in S207, (Td,0,j,L) is stored as the data for the Update Process, unlike S107. That is, to remember that this decision is based on the integration of the N Collection Units 21, the second value of the data is stored as 0 (an invalid Collection Unit Number).

Next, in S202, the Infection Quantification Function 3 is called. In its step S2021, qi'[t] and ei'[t] (0≤t≤Td, 1≤i≤N) are calculated for all N Collection Units 21, and after obtaining ei"[t] as mentioned earlier, their weighted average is determined as e0"[t] (0≤t≤Td). Note that, in this example, ei"[t] is determined as ei"[t]=ei'[t]/ei'[0], but other methods are also acceptable.

In the other steps S203, S204, S205, and S206, as in Figure 6, the entries of the Decision Criteria 41 are examined sequentially from the beginning (j=1). When an entry j where the Condition is true is found, the Countermeasure L written there is output. And in S204, the "Conditions" of the Decision Criteria 41 may reference Td, e0", EQ", and Environmental Data as appropriate (other references, such as ei' and Ei for 1≤i≤N, are not prohibited, but are not used in this example).

Additionally, from the Decision Criteria 41 with 3 entries illustrated in Embodiment 1, the example of Decision Criteria 41 for the present Embodiment can be obtained by replacing qi' with e0" and Qi' with E0".

<Summary of the Present Embodiment>

As described above, by installing multiple Collection Units 21 in Area 6 and integrating their measurement results, Infection Countermeasures can be implemented based on more reliable information compared to the case where only a single unit is installed.

In the above discussion, the Average Relative Emission Quantity e0" was determined by integrating the Relative Emission Quantities ei'. Also, when the amount of people in each Observation Region Ri (1≤i≤N) is similar, the Relative Suspension Quantities qi' can similarly be converted into mutually comparable quantities. Then, their average (hereinafter referred to as the "Average Relative Suspension Quantity") can be determined as q0", which may also be referenced in the Decision Criteria 41. By doing so, the accuracy of Infection Countermeasures can be improved by integrating a broader range of information using Relative Suspension Quantities as well. Additionally, since the Average Relative Suspension Quantity q0" can be determined solely from the measured values VAi without measuring the Residence Quantity si', it allows for Infection Countermeasures that take less time and effort compared to using the Average Relative Emission Quantity e0".

Similarly, for the Relative Absorption Quantity ai', when the amount of people in each Observation Region Ri is similar, ai' can be converted into a mutually comparable quantity. Then, their average (hereinafter referred to as the "Average Relative Absorption Quantity") can be determined as a0", which may also be referenced in the Decision Criteria 41. By doing so, the accuracy of Infection Countermeasures can be improved by integrating a broader range of information using Relative Absorption Quantities as well.

Moreover, the following applications can also be considered. Taking the Average Relative Emission Quantity e0" as an example, it enables the estimation of the Relative Absorption Quantity a' within any region R (Collection Units 21 may not be installed in R)-where the Infection Situation is similar to that of each Observation Region Ri ($1 \le i \le N$)-by using the equation a'=e0"*s'*s" after determining the Relative Residence Quantities s' and s" for the people within R. That is, even in regions where Collection Units 21 are not installed, by measuring s' and s", a' can be estimated using e0". As briefly mentioned in the summary of Embodiment 2, this implies that if R is considered as the entire Area 6, by determining ei' or e0" from qi' within the smaller Observation Regions Ri ($1 \le i \le N$), a' can be estimated by measuring s' and s" within Area 6 and Infection Countermeasures can be implemented for the entire Area 6.

[Embodiment 4]

In the present Embodiment, a method for optimizing the process of Decision Unit 32 is described by extending Embodiment 1 (Embodiments 2 and 3 can be similarly extended). For Decision Process 1 of Embodiment 1, since there are a variety of methods for Infection Countermeasures, a general framework is presented, and one example is explained. The present Embodiment also does not focus on indicating which of the various optimization methods is preferable. Therefore, we again present a general framework and explain a simplified optimization method as an example.

As in Embodiment 1, we consider one Collection Unit (i=1 only) and Pathogen Type A as the only type of Propagating Substance. It is preferable to assume that A is also absent in environments where no infected people are present.

In the previous Embodiments, the Decision Unit 32 has output values such as L=20 or L=0 as Countermeasures. Here, the effectiveness of the Countermeasures will be verified at a later date, and optimization will be performed by increasing the value of L if the effectiveness is smaller than expected, or decreasing the value of L if it is larger. In this Embodiment, the effectiveness is verified using the Relative Emission Quantity ei', but it can also be modified to use the Relative Suspension Quantity qi' or the Relative Absorption Quantity ai' (Note that qi' and ai' differ from ei' in that they increase as the amount of people in the Observation Region Ri increases). Additionally, for the sake of simplicity in the explanation, it is assumed that the early termination of the lockdown will not be conducted.

Assume that, when the Decision Unit 32 executed the Decision Process at the past Decision Time Td, the 4 values saved in the last step S107 were (Td,i,j,L), and L>0. Then, a lockdown will begin for L days starting from time Td+1 and will end at time Td+L. The update process is executed at an appropriate time thereafter (for example, the day after the lockdown ends, namely at time Td+L+1), and at that time, the 4 saved values are passed as arguments.

<Update Process>

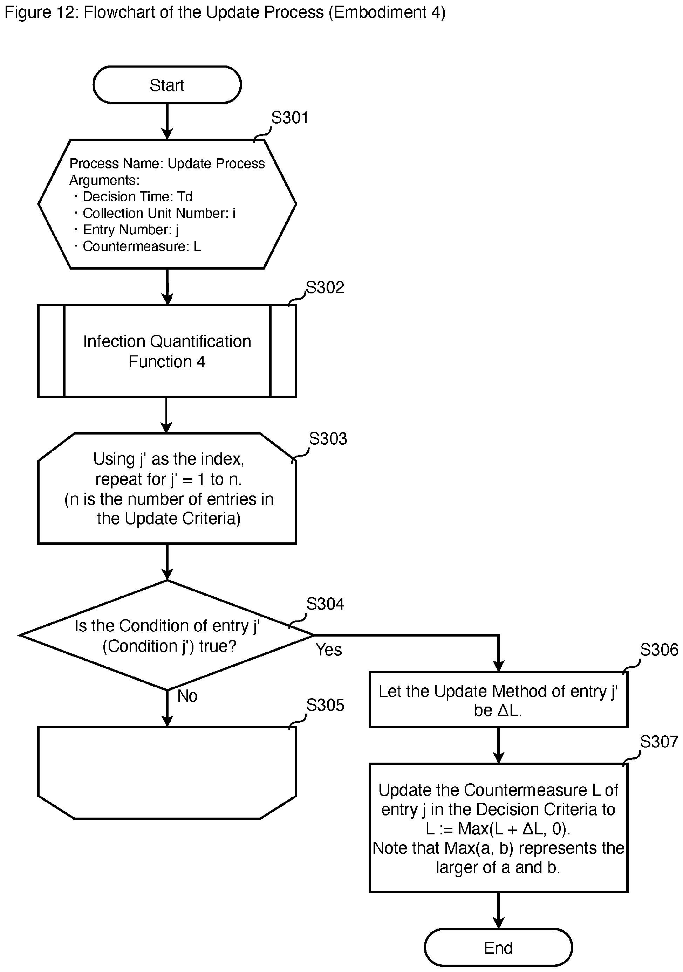

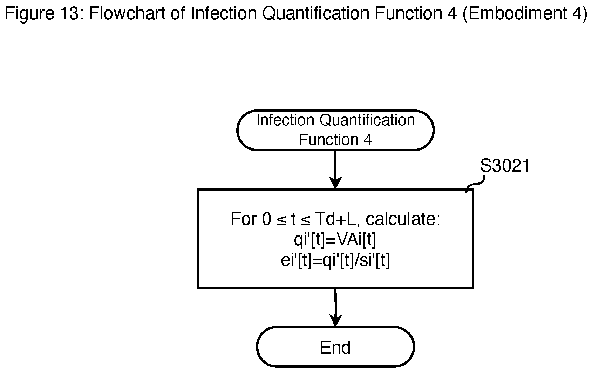

The Update Process will be explained using the flowchart in Figure 12. This Update Process calls the Infection Quantification Function 4 in Figure 13 as a subroutine. Note that the following symbols will be used below: measured value VAi, Relative Suspension Quantity qi', Relative Emission Quantity ei', Relative Residence Quantity si', Suspension Threshold Qi', and Emission Threshold Ei'. To ensure accuracy, brackets [] will be included without omission.

The Update Process starts with the arguments of Decision Time: Td, Collection Unit Number: i, Entry Number: j, and Countermeasure: L (S301). Next, the Infection Quantification Function 4 is called (S302). Where, for times t satisfying $0 \le t \le Td+L$, qi'[t]=VAi[t] and ei'[t]=qi'[t]/si'[t] are calculated (S3021). Next, using j' as an index, repeat the following from the first entry (j'=1) of the Update Criteria 42 to the last entry (j'=n, with n as the number of entries in the Update Criteria 42) (S303). First, check whether the Condition of entry j' (Condition j') is true. At this point, it is permissible to refer to Td, i, j, L, qi', ei', Qi', Ei', and Environmental Data from the Condition as needed (S304). If Condition j' is true, let the Update Method for that entry be ΔL (S306), and then update the Countermeasure L of entry j in the Decision Criteria 41 to Max(L+ΔL,0) (where Max(a,b) represents a if a=b, and otherwise the larger of a and b). Note that this expression means adding ΔL to L, but if L becomes a negative value, it will be assigned a value of 0 (S307). On the other hand, if Condition j' is false, return to the beginning of the loop (S305).

For example, consider the continuation of the example Decision Process in Embodiment 1. Assume that, for example, the 4 values (Td, i, j=1, L=20) were saved at this time. Also, let r=ei'[Td+L]/ei'[Td], and assume that the Update Criteria 42 consists of the following 3 entries: (1) Condition="r<0.3", Update Method ∆L=-1. (2) Condition="r≥0.6", Update Method ∆L=+1. (3) Update Sentinel.

In this case, if the Update Process is executed and r<0.3, it will reduce the Countermeasure L of entry j in the Decision Criteria 41 to 19, assuming that the lockdown period was too long. Additionally, if r≥0.6, it will increase L to 21, assuming that the lockdown period was too short. Otherwise, L will remain at 20.

<Summary of the Present Embodiment>

As mentioned above, by rewriting the Decision Criteria 41 through the Update Process, it is possible to gradually improve the initially adopted method of Infection Control, even if it is not optimal, to make it more optimal. Therefore, even if the information regarding the nature of the pathogen is insufficient, Infection Control can be initiated first and gradually improved while in operation. Additionally, even if the situation changes over time (for example, if the nature of the pathogen changes, leading to an increase or decrease in infectiousness, or if the number of people acquiring antibodies in the group increases, etc.), Infection Control can be optimally maintained by following those changes.

[Embodiment 5]

In general, assume that a quantity Ii (hereinafter referred to as "Indicator"), which represents the Infection Situation or is useful for Infection Countermeasures, is expressed as Ii[t]=Ii(t; VAi,si',si"; x,y,...; ui,vi,...) by a function that takes time t, measured value VAi, Relative Residence Quantities si' and si", Environmental Data x,y,..., and parameters ui,vi,... as arguments (note that the semicolon indicates grouping of arguments and serves as a mere separator like a comma). Here, the Environmental data x,y,..., and the parameters ui,vi,... are constants or time-varying quantities. Additionally, the function Ii can be expressed as a specific formula, or it can be realized through a computer simulation that outputs the result when the arguments are input.

In the previous Embodiments, as Indicators, we have used the Suspension Quantity qi, Relative Suspension Quantity qi', Emission Quantity ei, Relative Emission Quantity ei', Absorption Quantity ai, Relative Absorption Quantity ai', the number of newly infected people pi, etc. For an indicator Ii represented by a more general function, if the parameters ui,vi,... can be identified through regression analysis or machine learning using the actual measured values of Ii, it is possible to estimate Ii[t].

For example, in Embodiment 2, we assumed that the amount of pathogens taken into the body is proportional to the probability of infection when determining pi. However, even if the probability is in a more complex relationship, as long as the function Ii is defined, pi can be estimated.

Furthermore, once the function Ii is defined, by solving Ii with respect to one of its arguments, for example the Environmental Data x, x in turn may be expressed as a function of the other arguments of the function Ii and Ii[t]. In other words, x can be determined inversely from Ii[t]. This allows, for example, for the estimation of the proportional coefficient (coefficient k in Equation FKX) between the amount of pathogens taken into the body and the infection probability from qi', si", and pi. Furthermore, by monitoring the temporal changes in this proportional coefficient, it is also possible to capture signs of changes in the situation (such as the emergence of new variants). In addition, it is also possible to estimate si' from qi' and ei', that is, to estimate the amount of people within the Observation Region Ri using the amount of pathogens in the air.

[Embodiment 6]

The Infection Situation of the group may temporarily worsen or improve depending on temporal attributes (such as the day of the week or time of day, like morning or afternoon). For example, on Monday, due to the previous day being a holiday, people may become fatigued soon at their work, resulting in a larger amount of pathogens emitted compared to other days of the week.

To implement Infection Control without being overly sensitive to temporary fluctuations, one possible approach is to control based not only on the value at time t but also, for example, on the average of the past week (for Relative Emission Quantity ei', the average of ei'[t-6], ei'[t-5], ..., ei'[t]). Alternatively, the Countermeasures can be output by taking into account the attributes of the time. For example, the previously explained values such as Suspension Threshold Qi', Emission Threshold Ei', and Absorption Threshold Ai' can be changed according to the day of the week (for instance, setting Ei' on Mondays higher than on other days). More generally, entries that take into account the attribute of the time (not limited to the Decision Time Td but also attributes of other times, such as the attribute of time Td-1) are added to the Decision Criteria 41 and Update Criteria 42.

[Embodiment 7]

According to observations from the inventor's bodily reactions, during the outbreak of the novel coronavirus in Japan from early 2020, the amount of pathogens in the air is smaller during early hours such as in the morning and around noon, and increases over time.

As Infection Control, when restricting going out while minimizing damage to the economy, one possible approach is to restrict partial time of day rather than to restrict for the entire 24 hours of the day. By utilizing the above characteristics, if the duration of the restrictions is the same, the effectiveness in the evening is higher than in the morning or at noon (since the Relative Absorption Quantity ai' is defined as ai'=ei'*si'*si", it naturally becomes smaller when ei' is smaller, assuming the amount of people remains the same. Here, ei' represents the Relative Emission Quantity, while si' and si" represent the Relative Residence Quantities).

[Embodiment 8]

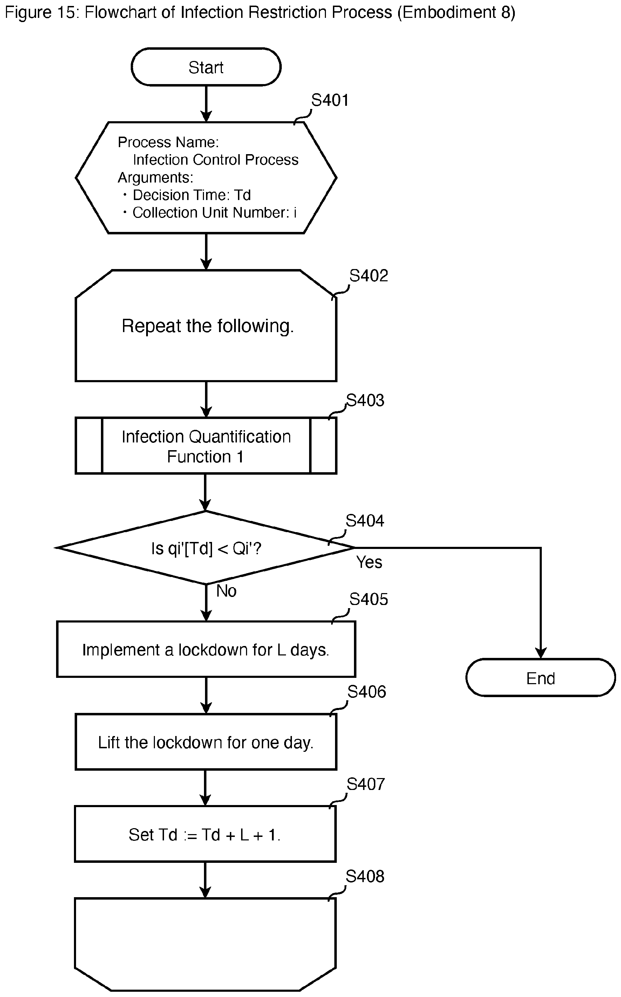

In the present Embodiment as well, we consider one Collection Unit 21 (i=1 only) and Pathogen Type A as the only type of Propagating Substance. In Figure 15, the Infection Control Process starts with the arguments of Decision Time: Td and Collection Unit number: i (S401). Subsequently, the following loop process is executed (S402). First, the Infection Quantification Function 1 is called to calculate the Relative Suspension Quantity qi' and the Relative Emission Quantity ei' (S403). Note that there is no necessity to call the Infection Quantification Function 1 here. It is also acceptable to call other functions such as Infection Quantification Function 2. Next, it judges whether the Infection Situation has sufficiently improved. Here, it checks whether qi'[Td] has become smaller than a threshold Qi', which is predetermined like the Suspension Threshold in Embodiment 1 (S404). Note that there is also no necessity to check the Relative Suspension Quantity qi'. It is acceptable to check the Relative Emission Quantity ei' or the Relative Absorption Quantity ai'. Additionally, it is also acceptable to examine complex conditions, such as the "Condition" in Decision Criteria 41, rather than simply comparing to the threshold. If the result of the judgement shows that the Infection Situation has sufficiently improved, the Infection Control Process terminates. Otherwise, a lockdown for L days is implemented (S405). Note that L is a positive value, and it can always be the same value (for example, L=3), or it can be changed each time the loop repeats. After that, the lockdown is lifted for just one day, and normal life is resumed (S406). Note that there is also no necessity for it to be one day, and an appropriate number of days, such as two, can be chosen. Finally, L+1 is added to Td so that the Infection Situation on the day when the lockdown was lifted in Step S406 can be calculated in the call to the Infection Quantification Function 1 in the next loop (S407).

By venturing to lift the lockdown for just one day during the lockdown, this Infection Control Process has the effect of resuming economic activities and making it easier to grasp the infection situation (by venturing to return the amount of going out to normal, thereby increasing the amount of virus in the air so that the measured value VAi reflects the infection situation of more people).

[DESCRIPTION OF SYMBOLS]

| 1 | Infection Countermeasure System |
| 2 | Observation Unit |
| 21 | Collection Unit |
| 22 | Measurement Unit |
| 3 | Analysis Unit |
| 31 | Memory Unit |
| 32 | Decision Unit |
| 33 | Update Unit |
| 41 | Decision Criteria |
| 42 | Update Criteria |
| 43 | Area Information |
| 5 | Analysis Device |
| 51 | Processing Device |
| 52 | Input Device |
| 53 | Output Device |
| 54 | Memory Device |
| 6 | Area |

**Claims**

1. An Infection Countermeasure System, comprising:

   an Observation Unit configured to collect a Propagating Substance (X) within a region Ri and measure the quantity thereof; and
   an Analysis Unit configured to calculate one or more Indicator values or approximate values thereof for Infection Countermeasures of the entire Area, based on the measured value (VXi) of the quantity of the Propagating Substance.

2. The Infection Countermeasure System according to Claim 1,

   wherein the Analysis Unit is configured to output Countermeasures for Infection Countermeasures of the entire Area,
   based on the calculated Indicator values or approximate values thereof.

3. The Infection Countermeasure System according to Claim 2,

   wherein the Analysis Unit is configured to input a Residence Quantity (si), which represents the cumulative duration of stay of a group within the region Ri,
   to estimate a Suspension Quantity (qi) of the Pathogen from the measured value (VXi) of the quantity of the Propagating Substance,
   to calculate an Emission Quantity (ei) or an Absorption Quantity (ai) of the Pathogen using the Suspension Quantity (qi) and the Residence Quantity (si),
   and to output the Countermeasures based on the results of the calculation.

4. The Infection Countermeasure System according to Claim 3,

wherein the Emission Quantity (ei) of the Pathogen is obtained based on a result of dividing the Suspension Quantity (qi) by the Residence Quantity (si).

5. The Infection Countermeasure System according to Claim 3,
wherein the Absorption Quantity (ai) of the Pathogen is obtained based on a result of multiplying the Suspension Quantity (qi) by the Residence Quantity (si).

6. A method for Infection Countermeasures, comprising:

a step of collecting a Propagating Substance (X) within a region Ri and measuring the quantity thereof;
a step of calculating one or more Indicator values or approximate values thereof for Infection Countermeasures of the entire Area, based on the measured value (VXi) of the quantity of the Propagating Substance; and
a step of outputting Countermeasures for Infection Countermeasures of the entire Area, based on the calculated Indicator values or approximate values thereof.

Figure 1: Functional Block Diagram of Infection Countermeasure System 1

Figure 2: Configuration Diagram of Analysis Unit 3

3

Analysis Device

5

51

## Processing Device

52

### Input Device

53

### Output Device

54

### Memory Device

·······

Output from
Observation
Devices

Countermeasures, etc.

Environmental data, etc.

Figure 3: Area 6 with one Collection Unit 21

Figure 4: Area with Multiple (N) Observation Regions

Figure 5: Decision Criteria

Decision Criteria

| | Condition | Countermeasure L |
|---|---|---|
| entry 1 | Condition 1 | 10 |
| entry 2 | Condition 2 | 15 |
| ⋮ | ⋮ | ⋮ |
| entry n-1 | Condition n-1 | 5 |
| entry n | always true | 0 |

Figure 6: Flowchart of Decision Process 1 (Embodiment 1)

```
                    ╭─────────────╮
                    │    Start    │
                    ╰─────────────╯
                           │
                           ▼              ⌐ S101
           ╱─────────────────────────────╲
          ╱  Process Name:                 ╲
         ⟨    Decision Process 1            ⟩
          ╲  Arguments:                    ╱
           ╲  · Decision Time: Td         ╱
            ╲ · Collection Unit Number: i╱
             ╲─────────────────────────╱
                           │
                           ▼              ⌐ S102
         ┌─┬─────────────────────────┬─┐
         │ │                         │ │
         │ │  Infection Quantification│ │
         │ │      Function 1         │ │
         │ │                         │ │
         └─┴─────────────────────────┴─┘
                           │
                           ▼              ⌐ S103
            ╱──────────────────────────╲
           │  Using j as the index,     │
           │  repeat for j = 1 to n.    │
           │  (n is the number of entries in│
           │  the Decision Criteria)    │
            ╲──────────────────────────╱
                           │
                           ▼
                         ╱─╲              ⌐ S104
                       ╱     ╲
                     ╱  Is the ╲
                   ⟨ Condition of entry j ⟩─────── Yes ───────┐
                     ╲(Condition j) true?╱                     │
                       ╲     ╱                                 │
                         ╲─╱                                   │
                          │                                    ▼          ⌐ S106
                         No                        ┌──────────────────────┐
                          │                         │ Output the           │
                          ▼              ⌐ S105     │ Countermeasure L of   │
           ╱──────────────────────────╲            │ entry j.             │
          │                            │            └──────────────────────┘
          │                            │                        │
           ╲──────────────────────────╱                        ▼          ⌐ S107
                                              ┌──────────────────────┐
                                              │ If the Update Process is│
                                              │ performed, save (Td,i,j,L)│
                                              │ in the Memory Unit.    │
                                              └──────────────────────┘
                                                          │
                                                          ▼
                                                   ╭─────────────╮
                                                   │     End     │
                                                   ╰─────────────╯
```

Figure 7: Flowchart of Infection Quantification Function 1 (Embodiment 1)

```
         ╭─────────────────────────╮
         │ Infection Quantification │
         │       Function 1         │
         ╰─────────────────────────╯
                      │
                      ▼
         ┌─────────────────────────┐ ── S1021
         │  For 0 ≤ t ≤ Td, calculate: │
         │      qi'[t]=VAi[t]       │
         │    ei'[t]=qi'[t]/si'[t]  │
         └─────────────────────────┘
                      │
                      ▼
         ╭─────────────────────────╮
         │          End            │
         ╰─────────────────────────╯
```

Figure 8: Flowchart of Infection Quantification Function 2 (Embodiment 2)

```
      ╭────────────────────────────╮
      │  Infection Quantification  │
      │        Function 2          │
      ╰────────────────────────────╯
                   │
                   ▼                        S1022
      ┌────────────────────────────┐
      │   For 0 ≤ t ≤ Td, calculate:│
      │      qi'[t]=VAi[t]          │
      │      ei'[t]=qi'[t]/si'[t]   │
      │      ai'[t]=qi'[t]*si''[t]  │
      └────────────────────────────┘
                   │
                   ▼
          ╭──────────────────╮
          │       End        │
          ╰──────────────────╯
```

Figure 9: Flowchart of Decision Process 3 (Embodiment 3)

```
                    ┌─────────────────┐
                    │      Start      │
                    └────────┬────────┘
                             │
                             ▼                    S201
        ╱───────────────────────────────────────╲
        │  Process Name: Decision Process 3      │
        │  Arguments:                            │
        │  · Decision Time: Td                   │
        ╲───────────────────────────────────────╱
                             │
                             ▼                    S202
        ┌─┬─────────────────────────────────────┬─┐
        │ │     Infection Quantification        │ │
        │ │          Function 3                 │ │
        └─┴─────────────────────────────────────┴─┘
                             │
                             ▼                    S203
        ┌─────────────────────────────────────────┐
        │         Using j as the index,            │
        │         repeat for j = 1 to n.           │
        │   (n is the number of entries in         │
        │        the Decision Criteria)            │
        └─────────────────────────────────────────┘
                             │
                             ▼
                      ╱─────────────╲              S204
                   ╱                  ╲
                 ╱  Is the Condition of ╲
                ⟨    entry j             ⟩────── Yes ──────┐
                 ╲  (Condition j) true? ╱                  │
                   ╲                  ╱                     │
                      ╲─────────────╱                       │
                             │                              ▼              S206
                             No                   ┌──────────────────────┐
                             ▼          S205       │ Output the Countermeasure L of │
        ┌──────────────────────────────┐          │        entry j.       │
        │                              │          └──────────┬───────────┘
        │                              │                     │
        └──────────────────────────────┘                     ▼              S207
                                                  ┌──────────────────────┐
                                                  │ If the Update Process is │
                                                  │ performed, save (Td,0,j,L) in the │
                                                  │       Memory Unit.    │
                                                  └──────────┬───────────┘
                                                             │
                                                             ▼
                                                  ┌──────────────────────┐
                                                  │         End          │
                                                  └──────────────────────┘
```

Figure 10: Flowchart of Infection Quantification Function 3 (Embodiment 3)

Infection Quantification
Function 3

S2021

For $1 \leq i \leq N$ and $0 \leq t \leq Td$, define:
$qi'[t]=VAi[t]$
$ei'[t]=qi'[t]/si'[t]$
$ei''[t]=ei'[t]/ei'[0]$
Also, let $W=(w1+w2+ \ldots +wN)$, and calculate:
$e0''[t]=(w1*e1''[t]+w2*e2''[t]+ \ldots +wN*eN''[t])/W$

End

Figure 11: Update Criteria

Update Criteria

|  | Condition | Update Method ΔL |
|---|---|---|
| entry 1 | Condition 1 | +1 |
| entry 2 | Condition 2 | 0 |
| ⋮ | ⋮ | ⋮ |
| entry n-1 | Condition n-1 | -2 |
| entry n | always true | 0 |

Figure 12: Flowchart of the Update Process (Embodiment 4)

Start

S301

Process Name: Update Process
Arguments:
· Decision Time: Td
· Collection Unit Number: i
· Entry Number: j
· Countermeasure: L

S302

Infection Quantification
Function 4

S303

Using j' as the index,
repeat for j' = 1 to n.
(n is the number of entries in
the Update Criteria)

S304

Is the Condition of entry j'
(Condition j') true?

Yes

No

S306

Let the Update Method of entry j'
be ΔL.

S305

S307

Update the Countermeasure L of
entry j in the Decision Criteria to
L := Max(L + ΔL, 0).
Note that Max(a, b) represents the
larger of a and b.

End

Figure 13: Flowchart of Infection Quantification Function 4 (Embodiment 4)

Infection Quantification
Function 4

S3021

For $0 \le t \le Td+L$, calculate:
$qi'[t]=VAi[t]$
$ei'[t]=qi'[t]/si'[t]$

End

Figure 14: Relationship Between the Number of Newly Infected People and the Number of Newly Confirmed People

Figure 15: Flowchart of Infection Restriction Process (Embodiment 8)

```
                    ⟮  Start  ⟯
                         │
                         ▼
                                              S401
        ╱──────────────────────────────╲
       ╱  Process Name:                  ╲
      │       Infection Control Process    │
      │   Arguments:                       │
       ╲    · Decision Time: Td           ╱
        ╲   · Collection Unit Number: i  ╱
         ╲──────────────────────────────╱
                         │
                         ▼
                                              S402
        ╱────────────────────────────╲
       │                              │
       │     Repeat the following.    │
       │                              │
       └──────────────────────────────┘
                         │
                         ▼
                                              S403
       ┌─┬──────────────────────────┬─┐
       │ │  Infection Quantification │ │
       │ │        Function 1         │ │
       └─┴──────────────────────────┴─┘
                         │
                         ▼
                                       S404
            ╱─────────────────────╲
           ╱                       ╲
          ⟨    Is qi'[Td] < Qi'?    ⟩──── Yes ──────┐
           ╲                       ╱                 │
            ╲─────────────────────╱                  │
                     │ No                            │
                     ▼                               │
                                       S405          │
       ┌──────────────────────────────┐              │
       │  Implement a lockdown for L days. │         │
       └──────────────────────────────┘              ▼
                     │                           ⟮  End  ⟯
                     ▼
                                       S406
       ┌──────────────────────────────┐
       │   Lift the lockdown for one day. │
       └──────────────────────────────┘
                     │
                     ▼
                                       S407
       ┌──────────────────────────────┐
       │     Set Td := Td + L + 1.     │
       └──────────────────────────────┘
                     │
                     ▼
                                       S408
       ╱──────────────────────────────╲
      │                                │
       ╲──────────────────────────────╱
```

Figure 16: Diagram Explaining an Advantage of Embodiment 2

## INTERNATIONAL SEARCH REPORT

| International application No. |
|---|
| **PCT/JP2023/034161** |

| A. | CLASSIFICATION OF SUBJECT MATTER |
|---|---|

***G16H 50/80*** (2018.01)i
FI:  G16H50/80

According to International Patent Classification (IPC) or to both national classification and IPC

| B. | FIELDS SEARCHED |
|---|---|

Minimum documentation searched (classification system followed by classification symbols)

G16H50/80

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2015-117950 A (PANASONIC IP MANAGEMENT CORP.) 25 June 2015 (2015-06-25) paragraphs [0013]-[0020], [0025], [0032], [0035], [0036]-[0039], [0046], [0049]-[0053] | 1-2, 6 |
| A | entire text, all drawings | 3-5 |
| Y | JP 2015-206700 A (PANASONIC IP MANAGEMENT CORP.) 19 November 2015 (2015-11-19) paragraphs [0064]-[0065], [0078]-[0079], [0087]-[0091], [0097], [0103]-[0104], [0141] | 1-2, 6 |
| A | entire text, all drawings | 3-5 |
| A | US 2020/0385296 A1 (BIOBOT ANALYTICS, INC.) 10 December 2020 (2020-12-10) entire text, all drawings | 1-6 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 October 2023** | **07 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/034161**

| Patent document cited in search report | | | Publication date (day/month/year) | Patent family member(s) | Publication date (day/month/year) |
|---|---|---|---|---|---|
| JP | 2015-117950 | A | 25 June 2015 | (Family: none) | |
| JP | 2015-206700 | A | 19 November 2015 | (Family: none) | |
| US | 2020/0385296 | A1 | 10 December 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020008969 A